(19) Europäisches Patentamt
European Patent Office
Office européen des brevets

(11) **EP 4 201 342 A1**

(12) **EUROPEAN PATENT APPLICATION**

(43) Date of publication:
**28.06.2023 Bulletin 2023/26**

(21) Application number: **22168632.2**

(22) Date of filing: **15.04.2022**

(51) International Patent Classification (IPC):
***A61B 8/08*** *(2006.01)*     ***A61B 8/12*** *(2006.01)*

(52) Cooperative Patent Classification (CPC):
**A61B 8/0891; A61B 8/12; A61B 8/5223**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.12.2021 US 202163292543 P**

(71) Applicant: **Koninklijke Philips N.V.
5656 AG Eindhoven (NL)**

(72) Inventors:
• **RAJGURU, Nikhil Sreedhar
Eindhoven (NL)**
• **NAIR, Anuja
Eindhoven (NL)**

(74) Representative: **Philips Intellectual Property & Standards
High Tech Campus 52
5656 AG Eindhoven (NL)**

(54) **INTRAVASCULAR ULTRASOUND IMAGING FOR CALCIUM DETECTION AND ANALYSIS**

(57) A system (100) includes a processor circuit (134) that receives the IVUS imaging data from the IVUS imaging device (102). The processor circuit identifies calcium (1023) based on the IVUS imaging data. The processor circuit determines, based on the identification of the calcium, at least one of: a distance (1044) between the calcium and the lumen border (1032); or a density of the calcium relative to the tissue. The processor circuit outputs a screen display to a display (132) in communication with the processor circuit. The screen display includes an IVUS image (1000) generated based on the IVUS imaging data; and at least one of: a visual representation of the distance; or a visual representation of the density.

FIG. 10

EP 4 201 342 A1

**Description**

**TECHNICAL FIELD**

The present disclosure relates generally to identifying and analyzing calcium within intravascular images in blood vessel.

**BACKGROUND**

Intraluminal imaging is widely used in interventional treatment of patient vasculature as a diagnostic tool for assessing a diseased vessel, such as an artery, within the human body to determine the need for treatment, select the proper treatment method, and assess its effectiveness. An intraluminal imaging device including one or more ultrasound transducers is passed into the vessel and guided to the area to be imaged. The transducers emit ultrasonic energy. Ultrasonic waves are partially reflected by discontinuities in tissue structures (such as various layers of the vessel wall), red blood cells, and other features of interest. Echoes from the reflected waves are received by the transducer and passed along to an intraluminal imaging system. The imaging system processes the received ultrasound echoes to produce a cross-sectional intravascular ultrasound (IVUS) image of the vessel where the device is placed.
To determine the location and extent of a diseased vessel, a physician generally analyzes the vessel based on the IVUS images received from the intraluminal imaging system. This analysis includes measurements made on the vessel that may help the physician to determine the blood flow at various locations along the vessel and include the diameter of the vessel and cross-sectional area of the vessel. An important step in the analysis of a vessel may be to assess the presence and extent of vessel blockages, including calcium deposits, within the vessel. Calcium is generally seen within a vessel with various other substances which may contribute to a blockage, such as other types of plaque build-up. The correct treatment method for a blockage depends on the amount of calcium within a blockage as opposed to other plaque, the depth of the calcium, the thickness of the calcium, how far the calcium extends along the vessel, and the distribution of calcium with other plaque including solid calcium regions or calcium interspersed with other plaque. When analyzing an IVUS image from a patient, an inexperienced physician may incorrectly identify calcium and other plaque within the vessel, leading to an incorrect treatment method decision. Even for experienced physicians, correctly identifying calcium and other plaque within an image and making and comparing measurements of calcium depth, thickness, and distribution across multiple IVUS images can be difficult and time-consuming.

**SUMMARY**

Embodiments of the present disclosure are systems, devices, and methods for identifying and analyzing calcium within intravascular images in arteries. In particular, a deep learning network is implemented to identify calcium and other plaque within the blood vessel, quantify the level of calcification shown in an image, and generate intuitive visual representations of calcium distribution to promote efficient and accurate treatment decisions by physicians. For example, a processor circuit may implement a deep learning network trained to identify calcium and other plaque within IVUS images. Calcium within an IVUS image is quantified according to various criteria. The depth of calcium within a blockage may be measured. The thickness of a particular calcium deposit may also be measured. The presence of calcium within adjacent IVUS images extending along an imaged vessel may be measured to determine the length of a calcium deposit as an indication of diffused disease. The distribution of calcium within the vessel may also be quantified to assist the physician in selecting an appropriate treatment method. The distribution of calcium with respect to other tissue types of plaque material may also be quantified as a relative measurment. A calcium arc identifying in which angular direction the calcium deposit is positioned may also be determined. Using these measurements, the processor circuit generates a calcium distribution ring around the IVUS image. Using different colors and patterns, the calcium distribution ring quickly identifies for the physician the location and extent of calcification shown in the image. The processor circuit also assigns a frame score to each IVUS image to describe the extent of calcification of the image. The calcium distribution rings and/or frame scores from multiple IVUS images may be displayed to a physician to give the physician a fast and accurate summary of the location and extent of calcium along a vessel to assist the physician in selecting the proper treatment method.
Embodiments of the disclosure include a system capable of analyzing the intravascular ultrasound properties of an IVUS image. These properties may not be easily visually interpretable. The system is capable of providing an automated analysis of the regions within the lumen and vessel boundaries. This can lead to a faster, easier, and more efficient workflow for the user of the system and the patient, and could potentially lead to improved outcomes for the patient.
In an exemplary aspect of the present disclosure, a system is provided. The system includes an IVUS imaging catheter configured to obtain IVUS imaging data while positioned within a blood vessel of a patient, wherein the blood vessel comprises: a wall formed of tissue comprising calcium; and a lumen border defining a lumen; and a processor circuit configured for communication with the IVUS imaging catheter, wherein the processor circuit is configured to: receive the IVUS imaging data from the IVUS imaging catheter; identify the calcium based on the IVUS imaging data; determine, based on the identification of the calcium, at least one of: a distance between the calcium and the lumen border; or a density of the calcium relative to the tissue; output a screen display to a display in communication with the processor circuit, wherein the screen display comprises: an IVUS image generated based on the IVUS imaging data; and at least one of: a visual representation of the distance; or a visual representation of the density.

In some aspects, the processor circuit is configured to use a deep learning network to identify the calcium. In some aspects, the processor circuit is configured to use the deep learning network to determine at least one of the distance or the density. In some aspects, the deep learning network is trained using a plurality of annotated IVUS images, and wherein each of the plurality of annotated IVUS images comprises an annotation identifying the calcium. In some aspects, the distance comprises a radial distance. In some aspects, the processor circuit is configured to determine the density for at least one of a scan line of the IVUS image, a sector of the IVUS image, an entirety of the IVUS image, or a plurality of IVUS images. In some aspects, the screen display further comprises a graphical overlay overlaid on the IVUS image, and wherein the graphical overlay provides a visual representation of the calcium. In some aspects, the tissue comprises a plurality of tissue types other than calcium, the graphical overlay provides a visual representation of the plurality of tissue types other than calcium, and the plurality of tissue types other than calcium are grouped together such that the graphical overlay only includes the visual representation of the calcium and the visual representation of the plurality of tissue types other than calcium. In some aspects, the processor circuit is configured to adjust a transparency of the graphical overlay. In some aspects, the processor circuit is configured to determine an individual numerical score representative of the calcium, wherein the individual numerical score is based on at least one of the distance or the density.

In an exemplary aspect of the present disclosure, a system is provided. The system includes an IVUS imaging catheter configured to obtain IVUS imaging data while positioned within a blood vessel of a patient, wherein the blood vessel comprises: a wall formed of tissue comprising calcium; and a lumen border defining a lumen; and a processor circuit configured for communication with the IVUS imaging catheter, wherein the processor circuit is configured to: receive the IVUS imaging data from the IVUS imaging catheter; identify the calcium based on the IVUS imaging data; calculate, based on the identification of the calcium, an individual numerical score representative of the calcium using: a distance between the calcium deposit and the lumen border; a thickness of the calcium deposit; an angle spanned by the calcium deposit; and a length of the calcium deposit; and output a screen display to a display in communication with the processor circuit, wherein the screen display comprises: an IVUS image generated based on the IVUS imaging data; and a visual representation of the individual numerical score.

In some aspects, the processor circuit is configured to use a deep learning network to identify the calcium. In some aspects, the processor circuit is configured to use the deep learning network to determine the distance, the thickness, the angle, and the length. In some aspects, the distance extends radially, wherein the thickness extends radially, wherein the angle extends circumferentially, and wherein the length extends longitudinally. In some aspects, the distance, the thickness, the angle, and the length are weighted based on a location of the calcium within the wall. In some aspects, the individual numerical score corresponds to at least one of a scan line of the IVUS image, a sector of the IVUS image, an entirety of the IVUS image, or a plurality of IVUS images. In some aspects, the screen display further comprises at least one of: an extraluminal image of the blood vessel including the visual representation of the individual numerical score; or a longitudinal image of the blood vessel including the visual representation of the individual numerical score. In some aspects, the processor circuit is further configured to: determine a predicted calcium distribution within the vessel after a treatment procedure has been performed; and generate a predicted numerical score representative based on the predicted calcium distribution. In some aspects, the processor circuit is further configured to: receive additional IVUS imaging data from the IVUS imaging catheter after a treatment procedure has been performed; identify calcium based on the additional IVUS imaging data; calculate, based on the identification of the calcium, an additional individual numerical score representative of the calcium; and output, to the screen display, a comparison of the individual numerical score to the additional individual numerical score. In some aspects, the comparison comprises a numerical value representative of a difference in cross-sectional area of the blood vessel before and after the treatment procedure.

Additional aspects, features, and advantages of the present disclosure will become apparent from the following detailed description.

BRIEF DESCRIPTION OF THE DRAWINGS

Illustrative embodiments of the present disclosure will be described with reference to the accompanying drawings, of which:

Fig. 1 is a schematic diagram of an intraluminal imaging and extracorporeal imaging system, according to aspects of the present disclosure.
Fig. 2 is a diagrammatic top view of an ultrasound imaging assembly in a flat configuration, according to aspects of the present disclosure.

Fig. 3 is a diagrammatic perspective view of the ultrasound imaging assembly shown in Fig. 2 in a rolled configuration around a support member, according to aspects of the present disclosure.

Fig. 4 is a diagrammatic cross-sectional side view of the ultrasound imaging assembly shown in Fig. 3, according to aspects of the present disclosure.

Fig. 5 is a schematic diagram of a processor circuit, according to aspects of the present disclosure.

Fig. 6 is a diagrammatic view of an intravascular ultrasound image of a vessel with an obstruction, according to aspects of the present disclosure.

Fig. 7 is a schematic diagram of a deep learning algorithm, according to aspects of the present disclosure.

Fig. 8 is a is a schematic diagram of a convolutional neural network (CNN) configuration, according to aspects of the present disclosure.

Fig. 9 is a diagrammatic view of an intravascular ultrasound image with an overlay, according to aspects of the present disclosure.

Fig. 10 is a diagrammatic view of an intravascular ultrasound image with an overlay including thickness and depth measurements, according to aspects of the present disclosure.

Fig. 11 is a diagrammatic view of an intravascular ultrasound image with an overlay including a calcium arc measurement, according to aspects of the present disclosure.

Fig. 12 is a diagrammatic view of an intravascular ultrasound image with an overlay including a calcium composition ring, according to aspects of the present disclosure.

Fig. 13 is a diagrammatic view of an intravascular ultrasound image with an overlay including a calcium composition ring, according to aspects of the present disclosure.

Fig. 14 is a diagrammatic view of multiple calcium composition rings, according to aspects of the present disclosure.

Fig. 15 is a diagrammatic view of an in-line digital display, according to aspects of the present disclosure.

Fig. 16 is a diagrammatic view of a graphical user interface including an intravascular ultrasound image, an angiography image, and an in-line digital display, according to aspects of the present disclosure.

Fig. 17 is a flow diagram for a method of identifying the location and extent of calcium plaque within a vessel and conveying associated measurements to a user, according to aspects of the present disclosure.

Fig. 18 is a flow diagram for a method of identifying the location and extent of calcium plaque within a vessel and conveying associated measurements to a user, according to aspects of the present disclosure.

## DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure and that features disclosed for the system are contemplated to be used in corresponding methods and vice versa. For the sake of brevity, however, the numerous iterations of these combinations will not be described separately.

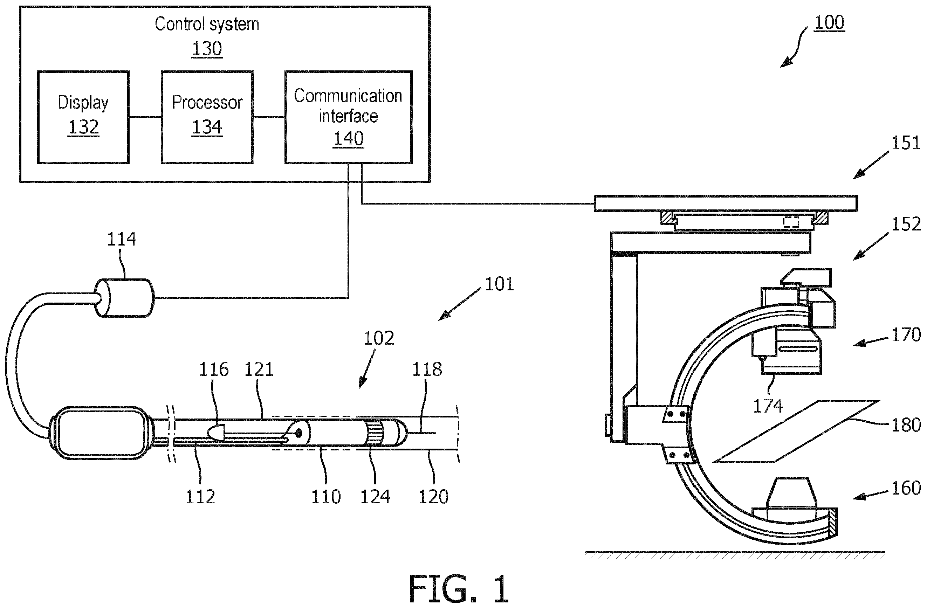

Fig. 1 is a schematic diagram of an intraluminal imaging and extracorporeal imaging system 100, according to aspects of the present disclosure. In some embodiments, the intraluminal imaging and extracorporeal imaging system 100 may include two separate systems or be a combination of two systems: an intraluminal sensing system 101 and an extracorporeal or extraluminal imaging system 151. The intraluminal sensing system 101 obtains medical data about a patient's body while the intraluminal device 102 is positioned inside the patient's body. For example, the intraluminal sensing system 101 can control the intraluminal device 102 to obtain intraluminal images of the inside of the patient's body while the intraluminal device 102 is inside the patient's body. The extracorporeal or extraluminal imaging system 151 obtains medical data about the patient's body while the extraluminal imaging device 152 is positioned outside the patient's body. For example, the extraluminal imaging system 151 can control extraluminal imaging device 152 to obtain extraluminal images of the inside of the patient's body while the extraluminal imaging device 152 is outside the patient's body.

The intraluminal imaging system 101 may be in communication with the extraluminal imaging system 151 through any suitable components. Such communication may be established through a wired cable, through a wireless signal, or by any other means. In addition, the intraluminal imaging system 101 may be in continuous communication with the extracorporeal imaging system 151 or may be in intermittent communication. For example, the two systems may be brought into temporary communication via a wired cable, or brought into communication via a wireless communication, or through any other suitable means at some point before, after, or during an examination. The extracorporeal or extraluminal imaging system may be one of an x-ray imaging system, a magnetic resonance imaging system, a computer tomograph, an extracorporeal ultrasound imaging system. In the following the exemplary embodiments are disclosed in detail for x-ray imaging system, however, in analogy, instead of the x-ray imaging system the use of any of the hereabove mentioned extracorporeal imaging systems is contemplated.

In addition, the intraluminal system 101 may receive data such as extracorporeal images, annotated x-ray images, metrics calculated with the x-ray imaging system 151, information regarding dates and times of examinations, types and/or severity of patient conditions or diagnoses, patient history or other patient information, or any suitable data or information from the x-ray imaging system 151. The x-ray imaging system 151 may also receive any of these data from the intraluminal imaging system 101. In some embodiments, and as shown in Fig. 1, the intraluminal imaging system 101 and the x-ray imaging system 151 may be in communication with the same control system 130. In this embodiment, both systems may be in communication with the same display 132, processor 134, and communication interface 140 shown as well as in communication with any other components implemented within the control system 130.

In some embodiments, the system 100 may not include a control system 130 in communication with the intraluminal imaging system 101 and the x-ray imaging system 151. Instead, the system 100 may include two separate control systems. For example, one control system may be in communication with or be a part of the intraluminal imaging system 101 and an additional separate control system may be in communication with or be a part of the x-ray imaging system 151. In this embodiment, the separate control systems of both the intraluminal imaging system 101 and the x-ray imaging system 151 may be similar to the control system 130. For example, each control system may include various components or systems such as a communication interface, processor, and/or a display. In this embodiment, the control system of the intraluminal imaging system 101 may perform any or all of the coregistration steps described in the present disclosure. Alternatively, the control system of the x-ray imaging system 151 may perform the coregistration steps described.

The intraluminal imaging system 101 can be an ultrasound imaging system. In some instances, the intraluminal imaging system 101 can be an intravascular ultrasound (IVUS) imaging system. The intraluminal imaging system 101 may include an intraluminal imaging device 102, such as a catheter, guide wire, or guide catheter, in communication with the control system 130. The control system 130 may include a display 132, a processor 134, and a communication interface 140 among other components. The intraluminal imaging device 102 can be an ultrasound imaging device. In some instances, the device 102 can be an IVUS imaging device, such as a solid-state IVUS device.

At a high level, the IVUS device 102 emits ultrasonic energy from a transducer array 124 included in a scanner assembly, also referred to as an IVUS imaging assembly, mounted near a distal end of the catheter device. The ultrasonic energy is reflected by tissue structures in the surrounding medium, such as a vessel 120, or another body lumen surrounding the scanner assembly 110, and the ultrasound echo signals are received by the transducer array 124. In that regard, the device 102 can be sized, shaped, or otherwise configured to be positioned within the body lumen of a patient. The communication interface 140 transfers the received echo signals to the processor 134 of the control system 130 where the ultrasound image (including flow information in some embodiments) is reconstructed and displayed on the display 132. The control system 130, including the processor 134, can be operable to facilitate the features of the IVUS imaging system 101 described herein. For example, the processor 134 can execute computer readable instructions stored on the non-transitory tangible computer readable medium.

The communication interface 140 facilitates communication of signals between the control system 130 and the scanner assembly 110 included in the IVUS device 102. This communication includes the steps of: (1) providing commands to integrated circuit controller chip(s) included in the scanner assembly 110 to select the particular transducer array element(s), or acoustic element(s), to be used for transmit and receive, (2) providing the transmit trigger signals to the integrated circuit controller chip(s) included in the scanner assembly 110 to activate the transmitter circuitry to generate an electrical pulse to excite the selected transducer array element(s), and/or (3) accepting amplified echo signals received from the selected transducer array element(s) via amplifiers included on the integrated circuit controller chip(s) of the scanner assembly 110. In some embodiments, the communication interface 140 performs preliminary processing of the echo data prior to relaying the data to the processor 134. In examples of such embodiments, the communication interface 140 performs amplification, filtering, and/or aggregating of the data. In an embodiment, the communication interface 140 also supplies high- and low-voltage DC power to support operation of the device 102 including circuitry within the scanner assembly 110.

The processor 134 receives the echo data from the scanner assembly 110 by way of the communication interface 140 and processes the data to reconstruct an image of the tissue structures in the medium surrounding the scanner assembly 110. The processor 134 outputs image data such that an image of the lumen 120, such as a cross-sectional image of the vessel 120, is displayed on the display 132. The lumen 120 may represent fluid filled or surrounded structures, both natural and man-made. The lumen 120 may be within a body of a patient. The lumen 120 may be a blood vessel, such as an artery or a vein of a patient's vascular system, including cardiac vasculature, peripheral vasculature, neural vasculature, renal vasculature, and/or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

In some embodiments, the IVUS device includes some features similar to traditional solid-state IVUS catheters, such as the EagleEye® catheter, Visions PV .014P RX catheter, Visions PV .018 catheter, Visions PV .035, and Pioneer Plus catheter, each of which are available from Koninklijke Philips N.V., and those disclosed in U.S. Patent No. 7,846,101 hereby incorporated by reference in its entirety. In some embodiments, the IVUS device may include some features similar to rotational IVUS catheter, such as the Revolution® 45 MHz rotational IVUS imaging catheter or the Refinity® short-tip rotational IVUS catheter, each of which are available from Koninklijke Philips N.V. For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors. It is understood that any suitable gauge wire can be used for the conductors. In an embodiment, the cable 112 can include a four-conductor transmission line arrangement with, e.g., 41 AWG gauge wires. In an embodiment, the cable 112 can include a seven-conductor transmission line arrangement utilizing, e.g., 44 AWG gauge wires. In some embodiments, 43 AWG gauge wires can be used.

The transmission line bundle 112 terminates in a patient interface module (PIM) connector 114 at a proximal end of the device 102. The PIM connector 114 electrically couples the transmission line bundle 112 to the communication interface 140 and physically couples the IVUS device 102 to the communication interface 140. In some embodiments, the communication interface 140 may be a PIM. In an embodiment, the IVUS device 102 further includes a guide wire exit port 116. Accordingly, in some instances the IVUS device 102 is a rapid-exchange catheter. The guide wire exit port 116 allows a guide wire 118 to be inserted towards the distal end to direct the device 102 through the vessel 120. It is noted that the intraluminal imaging system 101 may include a system of any suitable imaging modalities. In some embodiments, the intraluminal imaging device 102 may acquire intravascular images of any suitable imaging modality, including optical coherence tomography (OCT) and intravascular photoacoustic (IVPA).

In some embodiments, the intraluminal device 102 is a pressure sensing device (e.g., pressure-sensing guidewire) that obtains intraluminal (e.g., intravascular) pressure data, and the intraluminal system 101 is an intravascular pressure sensing system that determines pressure ratios based on the pressure data, such as fractional flow reserve (FFR), instantaneous wave-free ratio (iFR), and/or other suitable ratio between distal pressure and proximal/aortic pressure (Pd/Pa). In some embodiments, the intraluminal device 102 is a flow sensing device (e.g., flow-sensing guidewire) that obtains intraluminal (e.g., intravascular) flow data, and the intraluminal system 101 is an intravascular flow sensing system that determines flow-related values based on the pressure data, such as coronary flow reserve (CFR), flow velocity, flow volume, etc.

The x-ray imaging system 151 may include an x-ray imaging apparatus or device 152 configured to perform x-ray imaging, angiography, fluoroscopy, radiography, venography, among other imaging techniques. The x-ray imaging system 151 can generate a single x-ray image (e.g., an angiogram or venogram) or multiple (e.g., two or more) x-ray images (e.g., a video and/or fluoroscopic image stream) based on x-ray image data collected by the x-ray device 152. The x-ray imaging device 152 may be of any suitable type, for example, it may be a stationary x-ray system such as a fixed c-arm x-ray device, a mobile c-arm x-ray device, a straight arm x-ray device, or a u-arm device. The x-ray imaging device 152 may additionally be any suitable mobile device. The x-ray imaging device 152 may also be in communication with the control system 130. In some embodiments, the x-ray system 151 may include a digital radiography device or any other suitable device.

The x-ray device 152 as shown in Fig. 1 includes an x-ray source 160 and an x-ray detector 170 including an input screen 174. The x-ray source 160 and the detector 170 may be mounted at a mutual distance. Positioned between the x-ray source 160 and the x-ray detector 170 may be an anatomy of a patient or object 180. For example, the anatomy of the patient (including the vessel 120) can be positioned between the x-ray source 160 and the x-ray detector 170.

The x-ray source 160 may include an x-ray tube adapted to generate x-rays. Some aspects of the x-ray source 160 may include one or more vacuum tubes including a cathode in connection with a negative lead of a high-voltage power source and an anode in connection with a positive lead of the same power source. The cathode of the x-ray source 160 may additionally include a filament. The filament may be of any suitable type or constructed of any suitable material, including tungsten or rhenium tungsten, and may be positioned within a recessed region of the cathode. One function of the cathode may be to expel electrons from the high voltage power source and focus them into a well-defined beam aimed at the anode. The anode may also be constructed of any suitable material and may be configured to create x-radiation from the emitted electrons of the cathode. In addition, the anode may dissipate heat created in the process of generating x-radiation. The anode may be shaped as a beveled disk and, in some embodiments, may be rotated via an electric motor. The cathode and anode of the x-ray source 160 may be housed in an airtight enclosure, sometimes referred to as an envelope.

In some embodiments, the x-ray source 160 may include a radiation object focus which influences the visibility of an image. The radiation object focus may be selected by a user of the system 100 or by a manufacture of the system 100 based on characteristics such as blurring, visibility, heat-dissipating capacity, or other characteristics. In some embodiments, an operator or user of the system 100 may switch between different provided radiation object foci in a point-of-care setting.

The detector 170 may be configured to acquire x-ray images and may include the input screen 174. The input screen 174 may include one or more intensifying screens configured to absorb x-ray energy and convert the energy to light. The light may in turn expose a film. The input screen 174 may be used to convert x-ray energy to light in embodiments in which the film may be more sensitive to light than x-radiation. Different types of intensifying screens within the image intensifier may be selected depending on the region of a patient to be imaged, requirements for image detail and/or patient exposure, or any other factors. Intensifying screens may be constructed of any suitable materials, including barium lead sulfate, barium strontium sulfate, barium fluorochloride, yttrium oxysulfide, or any other suitable material. The input screen 374 may be a fluorescent screen or a film positioned directly adjacent to a fluorescent screen. In some embodiments, the input screen 374 may also include a protective screen to shield circuitry or components within the detector 370 from the surrounding environment. In some embodiments, the x-ray detector 170 may include a flat panel detector (FPD). The detector 170 may be an indirect conversion FPD or a direct conversion FPD. The detector 170 may also include charge-coupled devices (CCDs). The x-ray detector 370 may additionally be referred to as an x-ray sensor.

The object 180 may be any suitable object to be imaged. In an exemplary embodiment, the object may be the anatomy of a patient. More specifically, the anatomy to be imaged may include chest, abdomen, the pelvic region, neck, legs, head, feet, a region with cardiac vasculature, or a region containing the peripheral vasculature of a patient and may include various anatomical structures such as, but not limited to, organs, tissue, blood vessels and blood, gases, or any other anatomical structures or objects. In other embodiments, the object may be or include man-made structures.

In some embodiments, the x-ray imaging system 151 may be configured to obtain x-ray images without contrast. In some embodiments, the x-ray imaging system 151 may be configured to obtain x-ray images with contrast (e.g., angiogram or venogram). In such embodiments, a contrast agent or x-ray dye may be introduced to a patient's anatomy before imaging. The contrast agent may also be referred to as a radiocontrast agent, contrast material, contrast dye, or contrast media. The contrast dye may be of any suitable material, chemical, or compound and may be a liquid, powder, paste, tablet, or of any other suitable form. For example, the contrast dye may be iodine-based compounds, barium sulfate compounds, gadolinium-based compounds, or any other suitable compounds. The contrast agent may be used to enhance the visibility of internal fluids or structures within a patient's anatomy. The contrast agent may absorb external x-rays, resulting in decreased exposure on the x-ray detector 170.

In some embodiments, the extraluminal imaging system 151 could be any suitable extraluminal imaging device, such as computed tomography (CT), magnetic resonance imaging (MRI) or extracorporeal ultrasound imaging.

When the control system 130 is in communication with the x-ray system 151, the communication interface 140 facilitates communication of signals between the control system 130 and the x-ray device 152. This communication includes providing control commands to the x-ray source 160 and/or the x-ray detector 170 of the x-ray device 152 and receiving data from the x-ray device 152. In some embodiments, the communication interface 140 performs preliminary processing of the x-ray data prior to relaying the data to the processor 134. In examples of such embodiments, the communication interface 140 may perform amplification, filtering, and/or aggregating of the data. In an embodiment, the communication interface 140 also supplies high- and low-voltage DC power to support operation of the device 152 including circuitry within the device.

The processor 134 receives the x-ray data from the x-ray device 152 by way of the communication interface 140 and processes the data to reconstruct an image of the anatomy being imaged. The processor 134 outputs image data such that an image is displayed on the display 132. In an embodiment in which the contrast agent is introduced to the anatomy of a patient and a venogram is to be generated, the particular areas of interest to be imaged may be one or more blood vessels or other section or part of the human vasculature. The contrast agent may identify fluid filled structures, both natural and/or man-made, such as arteries or veins of a patient's vascular system, including cardiac vasculature, peripheral vasculature, neural vasculature, renal vasculature, and/or any other suitable lumen inside the body. For example, the x-ray device 152 may be used to examine any number of anatomical locations and tissue types, including without limitation all the organs, fluids, or other structures or parts of an anatomy previously mentioned. In addition to natural structures, the x-ray device 152 may be used to examine man-made structures such as any of the previously mentioned structures.

The processor 134 may be configured to receive an x-ray image that was stored by the x-ray imaging device 152 during a clinical procedure. The images may be further enhanced by other information such as patient history, patient record, IVUS imaging, pre-operative ultrasound imaging, pre-operative CT, or any other suitable data.

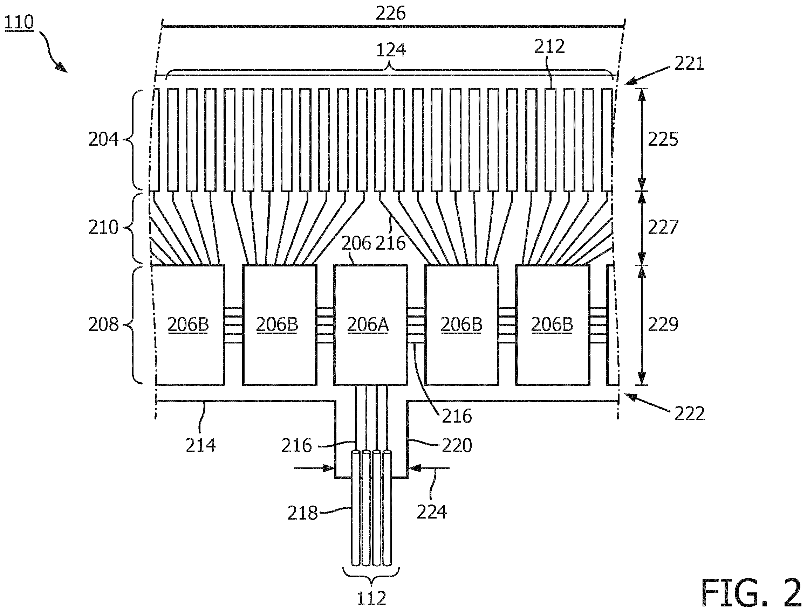

Fig. 2 is a diagrammatic top view of a portion of a flexible assembly 110, according to aspects of the present disclosure. The flexible assembly 110 includes a transducer array 124 formed in a transducer region 204 and transducer control logic dies 206 (including dies 206A and 206B) formed in a control region 208, with a transition region 210 disposed therebetween. The transducer array 124 includes an array of ultrasound transducer elements 212. The transducer control logic dies 206 are mounted on a flexible substrate 214 into which the transducer elements 212 have been previously integrated. The flexible substrate 214 is shown in a flat configuration in Fig. 2. Though six control logic dies 206 are shown in Fig. 2, any number of control logic dies 206 may be used. For example, one, two, three, four, five, six, seven, eight, nine, ten, or more control logic dies 206 may be used.

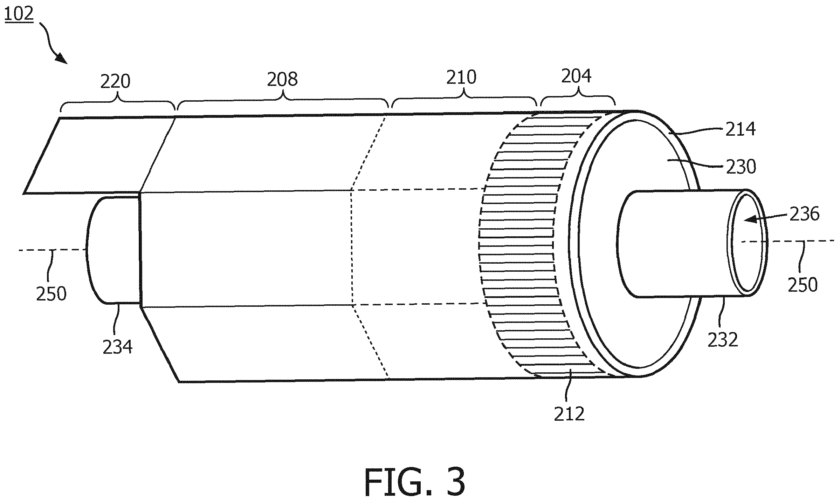

The flexible substrate 214, on which the transducer control logic dies 206 and the transducer elements 212 are mounted, provides structural support and interconnects for electrical coupling. The flexible substrate 214 may be constructed to include a film layer of a flexible polyimide material such as KAPTON™ (trademark of DuPont). Other suitable materials include polyester films, polyimide films, polyethylene napthalate films, or polyetherimide films, liquid crystal polymer, other flexible printed semiconductor substrates as well as products such as Upilex® (registered trademark of Ube Industries) and TEFLON® (registered trademark of E.I. du Pont). In the flat configuration illustrated in Fig. 2, the flexible substrate 214 has a generally rectangular shape. As shown and described herein, the flexible substrate 214 is configured to be wrapped around a support member 230 (Fig. 3) in some instances. Therefore, the thickness of the film layer of the flexible substrate 214 is generally related to the degree of curvature in the final assembled flexible assembly 110. In some embodiments, the film layer is between 5 $\mu$m and 100 $\mu$m, with some particular embodiments being between 5 $\mu$m and 25.1 $\mu$m, e.g., 6 $\mu$m.

The set of transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for cable 112, between a processing system, e.g., processing system 106, and the flexible assembly 110. Accordingly, the master control circuit may include control logic that decodes control signals received over the cable 112, transmits control responses over the cable 112, amplifies echo signals, and/or transmits the echo signals over the cable 112. The remaining controllers are slave controllers 206B. The slave controllers 206B may include control logic that drives a plurality of transducer elements 512 positioned on a transducer element 212 to emit an ultrasonic signal and selects a transducer element 212 to receive an echo. In the depicted embodiment, the master controller 206A does not directly control any transducer elements 212. In other embodiments, the master controller 206A drives the same number of transducer elements 212 as the slave controllers 206B or drives a reduced set of transducer elements 212 as compared to the slave controllers 206B. In an exemplary embodiment, a single master controller 206A and eight slave controllers 206B are provided with eight transducers assigned to each slave controller 206B. To electrically interconnect the control logic dies 206 and the transducer elements 212, in an embodiment, the flexible substrate 214 includes conductive traces 216 formed in the film layer that carry signals between the control logic dies 206 and the transducer elements 212. In particular, the conductive traces 216 providing communication between the control logic dies 206 and the transducer elements 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 112 when the conductors 218 of the cable 112 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 $\mu$m. For example, in an embodiment, 5 $\mu$m conductive traces 216 are separated by 5 $\mu$m of space. The width of a conductive trace 216 on the flexible substrate may be further determined by the width of the conductor 218 to be coupled to the trace or pad.

The flexible substrate 214 can include a conductor interface 220 in some embodiments. The conductor interface 220 can be in a location of the flexible substrate 214 where the conductors 218 of the cable 112 are coupled to the flexible substrate 214. For example, the bare conductors of the cable 112 are electrically coupled to the flexible substrate 214 at the conductor interface 220. The conductor interface 220 can be tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN®), polyether ether ketone (PEEK), nylon, Liquid Crystal Polymer (LCP), and/or other suitable materials.

Fig. 3 illustrates a perspective view of the scanner assembly 110 in a rolled configuration. In some instances, the flexible substrate 214 is transitioned from a flat configuration (Fig. 2) to a rolled or more cylindrical configuration (Fig. 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE," each of which is hereby incorporated by reference in its entirety. Depending on the application and embodiment of the presently disclosed invention, transducer elements 212 may be piezoelectric transducers, single crystal transducer, or PZT (lead zirconate titanate) transducers. In other embodiments, the transducer elements of transducer array 124 may be flexural transducers, piezoelectric micromachined ultrasonic transducers (PMUTs), capacitive micromachined ultrasonic transducers (CMUTs), or any other suitable type of transducer element. In such embodiments, transducer elements 212 may comprise an elongate semiconductor material or other suitable material that allows micromachining or similar methods of disposing extremely small elements or circuitry on a substrate.

In some embodiments, the transducer elements 212 and the controllers 206 can be positioned in an annular configuration, such as a circular configuration or in a polygon configuration, around a longitudinal axis 250 of a support member 230. It is understood that the longitudinal axis 250 of the support member 230 may also be referred to as the longitudinal axis of the scanner assembly 110, the flexible elongate member 121, or the device 102. For example, a cross-sectional profile of the imaging assembly 110 at the transducer elements 212 and/or the controllers 206 can be a circle or a polygon. Any suitable annular polygon shape can be implemented, such as one based on the number of controllers or transducers, flexibility of the controllers or transducers, etc. Some examples may include a pentagon, hexagon, heptagon, octagon, nonagon, decagon, etc. In some examples, the transducer controllers 206 may be used for controlling the ultrasound transducers 512 of transducer elements 212 to obtain imaging data associated with the vessel 120.

The support member 230 can be referenced as a unibody in some instances. The support member 230 can be composed of a metallic material, such as stainless steel, or a non-metallic material, such as a plastic or polymer as described in U.S. Provisional Application No. 61/985,220, "Pre-Doped Solid Substrate for Intravascular Devices," filed April 28, 2014, the entirety of which is hereby incorporated by reference herein. In some embodiments, support member 230 may be composed of 303 stainless steel. The support member 230 can be a ferrule having a distal flange or portion 232 and a proximal flange or portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process or a micro injection molding process.

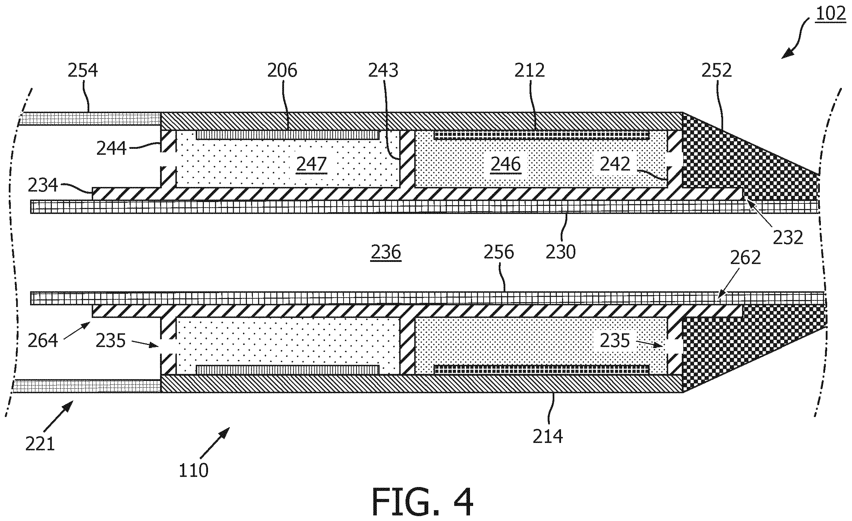

Referring now to Fig. 4, shown therein is a diagrammatic cross-sectional side view of a distal portion of the intraluminal imaging device 102, including the flexible substrate 214 and the support member 230, according to aspects of the present disclosure. The lumen 236 may be connected with the entry/exit port 116 and is sized and shaped to receive the guide wire 118 (Fig. 1). In some embodiments, the support member 230 may be integrally formed as a unitary structure, while in other embodiments the support member 230 may be formed of different components, such as a ferrule and stands 242, 243, and 244, that are fixedly coupled to one another. In some cases, the support member 230 and/or one or more components thereof may be completely integrated with inner member 256. In some cases, the inner member 256 and the support member 230 may be joined as one, e.g., in the case of a polymer support member. Stands 242, 243, and 244 that extend vertically are provided at the distal, central, and proximal portions respectively, of the support member 230. The stands 242, 243, and 244 elevate and support the distal, central, and proximal portions of the flexible substrate 214. In that regard, portions of the flexible substrate 214, such as the transducer portion 204 (or transducer region 204), can be spaced from a central body portion of the support member 230 extending between the stands 242, 243, and 244. The stands 242, 243, 244 can have the same outer diameter or different outer diameters. For example, the distal stand 242 can have a larger or smaller outer diameter than the central stand 243 and/or proximal stand 244 and can also have special features for rotational alignment as well as control chip placement and connection.

To improve acoustic performance, the cavity between the transducer array 212 and the surface of the support member 230 may be filled with an acoustic backing material 246. The liquid backing material 246 can be introduced between the flexible substrate 214 and the support member 230 via passageway 235 in the stand 242, or through additional recesses as will be discussed in more detail hereafter. The backing material 246 may serve to attenuate ultrasound energy emitted by the transducer array 212 that propagates in the undesired, inward direction.

The cavity between the circuit controller chips 206 and the surface of the support member 230 may be filled with an underfill material 247. The underfill material 247 may be an adhesive material (e.g. an epoxy) which provides structural support for the circuit controller chips 206 and/or the flexible substrate 214. The underfill 247 may additionally be any suitable material.

In some embodiments, the central body portion of the support member can include recesses allowing fluid communication between the lumen of the unibody and the cavities between the flexible substrate 214 and the support member 230. Acoustic backing material 246 and/or underfill material 247 can be introduced via the cavities (during an assembly process, prior to the inner member 256 extending through the lumen of the unibody. In some embodiments, suction can be applied via the passageways 235 of one of the stands 242, 244, or to any other suitable recess while the liquid backing material 246 is fed between the flexible substrate 214 and the support member 230 via the passageways 235 of the other of the stands 242, 244, or any other suitable recess. The backing material can be cured to allow it to solidify and set. In various embodiments, the support member 230 includes more than three stands 242, 243, and 244, only one or two of the stands 242, 243, 244, or none of the stands. In that regard the support member 230 can have an increased diameter distal portion 262 and/or increased diameter proximal portion 264 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions of the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can comprise a flexible elongate member. The proximal inner member 256 can be received within a proximal flange 234. The proximal outer member 254 abuts and is in contact with the proximal end of flexible substrate 214. A distal tip member 252 is coupled to the distal portion 262 of the support member 230. For example, the distal member 252 is positioned around the distal flange 232. The tip member 252 can abut and be in contact with the distal end of flexible substrate 214 and the stand 242. In other embodiments, the proximal end of the tip member 252 may be received within the distal end of the flexible substrate 214 in its rolled configuration. In some embodiments there may be a gap between the flexible substrate 214 and the tip member 252. The distal member 252 can be the distal-most component of the intraluminal imaging device 102. The distal tip member 252 may be a flexible, polymeric component that defines the distal-most end of the imaging device 102. The distal tip member 252 may additionally define a lumen in communication with the lumen 236 defined by support member 230. The guide wire 118 may extend through lumen 236 as well as the lumen defined by the tip member 252.

One or more adhesives can be disposed between various components at the distal portion of the intraluminal imaging device 102. For example, one or more of the flexible substrate 214, the support member 230, the distal member 252, the proximal inner member 256, the transducer array 212, and/or the proximal outer member 254 can be coupled to one another via an adhesive. Stated differently, the adhesive can be in contact with e.g. the transducer array 212, the flexible substrate 214, the support member 230, the distal member 252, the proximal inner member 256, and/or the proximal outer member 254, among other components.

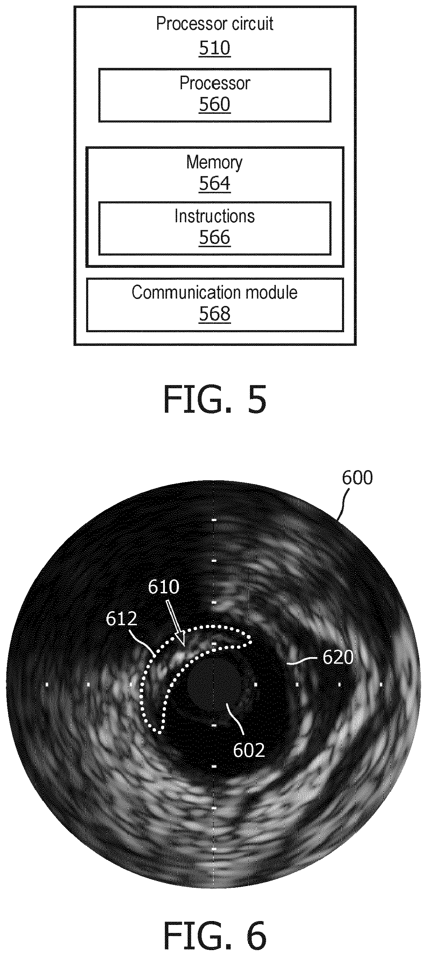

Fig. 5 is a schematic diagram of a processor circuit, according to aspects of the present disclosure. The processor circuit 510 may be implemented in the control system 130 of Fig. 1, the intraluminal imaging system 101, and/or the x-ray imaging system 151, or any other suitable location. In an example, the processor circuit 510 may be in communication with intraluminal imaging device 102, the x-ray imaging device 152, the display 132 within the system 100. The processor circuit 510 may include the processor 134 and/or the communication interface 140 (Fig. 1). One or more processor circuits 510 are configured to execute the operations described herein. As shown, the processor circuit 510 may include a processor 560, a memory 564, and a communication module 568. These elements may be in direct or indirect communication with each other, for example via one or more buses.

The processor 560 may include a CPU, a GPU, a DSP, an application-specific integrated circuit (ASIC), a controller, an FPGA, another hardware device, a firmware device, or any combination thereof configured to perform the operations described herein. The processor 560 may also be implemented as a combination of computing devices, e.g., a combination of a DSP and a microprocessor, a plurality of microprocessors, one or more microprocessors in conjunction with a DSP core, or any other such configuration.

The memory 564 may include a cache memory (e.g., a cache memory of the processor 560), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 564 includes a non-transitory computer-readable medium. The memory 564 may store instructions 566. The instructions 566 may include instructions that, when executed by the processor 560, cause the processor 560 to perform the operations described herein with reference to the probe 110 and/or the host 130 (Fig. 1). Instructions 566 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 568 can include any electronic circuitry and/or logic circuitry to facilitate direct or indirect communication of data between the processor circuit 510, the probe 110, and/or the display 132 and/or display 132. In that regard, the communication module 568 can be an input/output (I/O) device. In some instances, the communication module 568 facilitates direct or indirect communication between various elements of the processor circuit 510 and/or the probe 110 (Fig. 1) and/or the host 130 (Fig. 1).

Fig. 6 is a diagrammatic view of an intravascular ultrasound image 600 of a vessel 620 with an obstruction 610, according to aspects of the present disclosure. The ultrasound image 600 shown may be obtained by an intravascular imaging device 602. The ultrasound imaging device 602 may be similar to the imaging device 102 described with reference to Figs. 1-4. While the imaging device may be of any suitable type or modality, in some embodiments, the imaging device 602 may acquire intravascular images and/or other data via ultrasound transmission. In such an embodiment, the imaging device 602 may capture data corresponding to the surface of the lumen including the inner surface of the vessel wall or the inner surface of various obstructions within the vessel, as well as data corresponding to structures beneath the lumen surface such as the wall of the vessel, anatomies outside the vessel, the contents of various obstructions within the vessel or any other data. This advantageously provides the user of the system 100 with additional information which may be used to select proper treatment methods, as will be discussed in more detail hereafter.

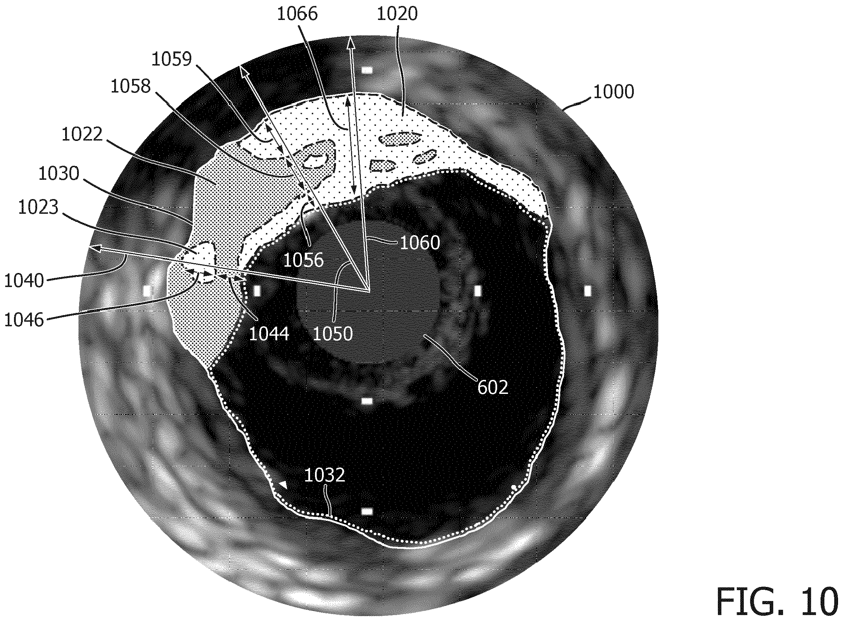

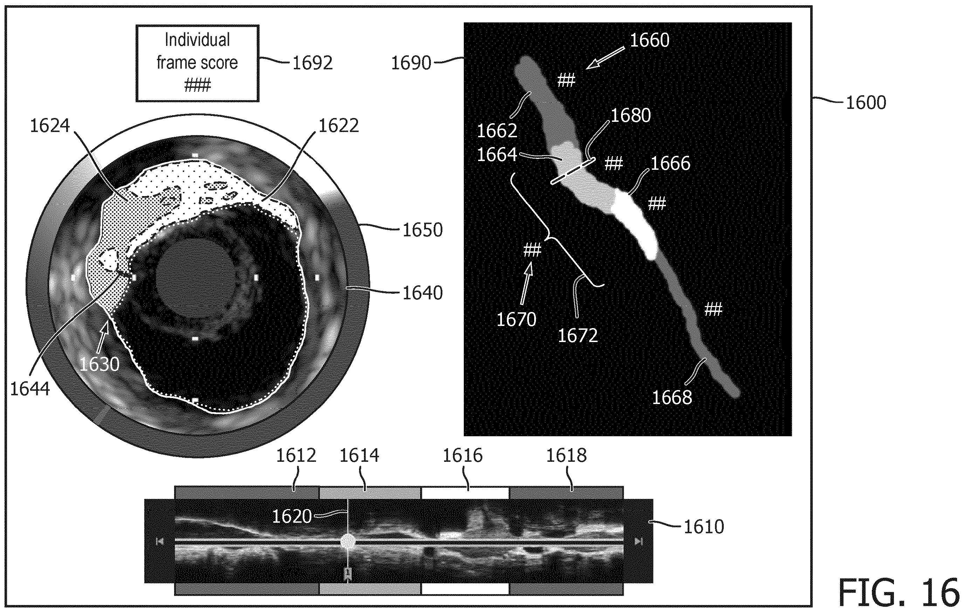

A region 612 is also shown in Fig. 6. In the image 600 shown, the region 612 may identify a location within the vessel 620 where plaque, or another type of obstruction is present. This obstruction 610 may restrict blood flow causing a variety of unpleasant or potentially dangerous conditions for the patient. As shown in the image 600, it may be difficult for a user of the system 100, particularly an inexperienced user, to determine what kind of obstruction is shown in an ultrasound image and to what extent the obstruction has grown or constricted blood flow. For example, an obstruction may include plaque, such as calcified deposits (e.g. calcium), dense calcium, fibrous tissues, fibro-fatty tissues, lipids, complex carbohydrates, necrotic core, various blood cells, dead blood cells, muscle cells, or other materials. An obstruction may include any or all of these materials in various compositions. The material of the obstruction and the composition of the obstruction may influence both the severity of the condition and immediacy of any need for treatment as well as the type of treatment recommended. The disclosed systems, devices, and methods advantageously identify the composition of various materials within obstructions in a vessel and succinctly convey this information to the user for enhanced diagnosis and treatment, as will be described in more detail hereafter. The obstruction may be referred to as a tissue. The tissue may include multiple tissue types other than calcium and the graphical overlay 1630 (Fig. 16) provides a visual representation 1624 (Fig. 16) of the multiple tissue types other than calcium. The other tissue types (e.g., 1624 of Fig. 16 and/or 1022 of Fig. 10) are grouped together such that the graphical overlay 1630 only includes a visual representation of the calcium and the visual representation of the multiple tissue types other than calcium.

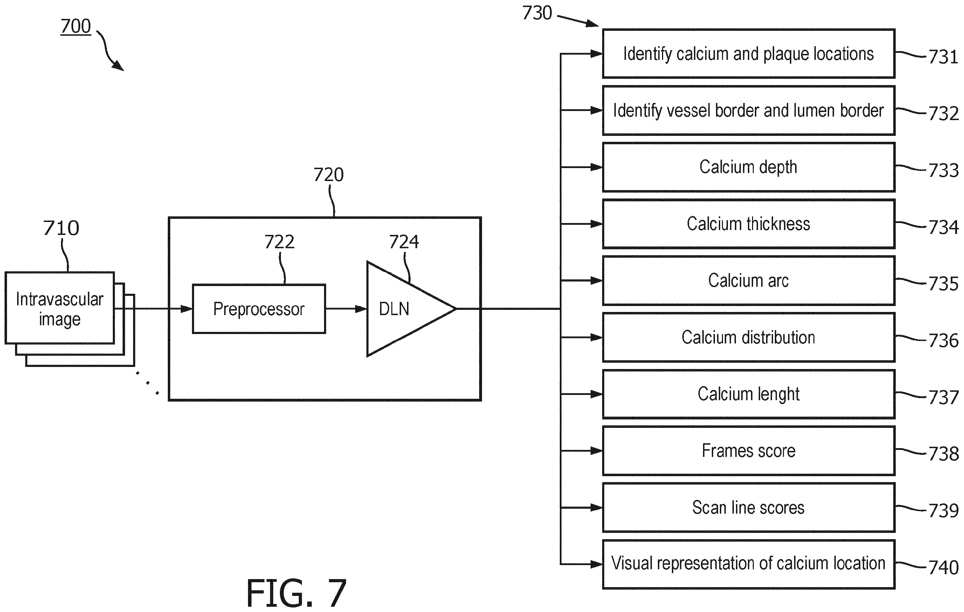

Fig. 7 is a schematic diagram of a machine learning algorithm 700, according to aspects of the present disclosure. The machine learning algorithm 700 can also be referred to as an artificial intelligence framework. In that regard, the artificial intelligence framework 700 may include a machine learning network and various aspects of the framework may be performed by the processor 134 and/or the processor circuit 510 and may include instructions similar to the instructions 566 previously described. The machine learning algorithm 700 may also be a deep learning algorithm in some embodiments. The processor circuit 510 may be configured to use a machine learning network to identify the calcium. The embodiment of the artificial intelligence framework 700 shown in Fig. 7 includes various input intravascular images 710. The intravascular images 710 may be received from various sources such as the intravascular imaging assembly 102, a picture archiving and communication system (PACS), or other image storage system. The intravascular images 710 may be received or arranged in a chronological order. For example, the intravascular images 710 may have been obtained by the imaging assembly 102 during a pullback procedure. The framework 700 may include an analysis module 720 with a preprocessor 722 and a deep learning network 724. In some embodiments, the analysis module 720 can be implemented in the host 130 (Fig. 1) and/or the processor circuit 510 (Fig. 5). The artificial intelligence framework 700 may generate multiple outputs 730.

The machine learning algorithm 700 may be trained to identify calcium and plaque locations 731, vessel borders and lumen borders 732, calcium depth 733, calcium thickness 734, a calcium arc 735, calcium distribution 736, calcium length 737, a frame score 738, scanline scores 739, and to generate a visual representation of calcium location 740. In some embodiments, the calcium arc 735 or any other identified metric may be a secondary metric derived from inputs such as calcium plaque locations 731, vessel borders and lumen borders 732, calcium depth 733, calcium thickness 734, etc. In some embodiments, the machine learning algorithm 700 may determine that multiple deposits of calcium identified within a particular IVUS image are to be included in the same calcium arc 735.

Training the machine learning algorithm 700 may be accomplished with various different techniques. In one embodiment, training the deep learning network may be accomplished by creating a large dataset of sample intravascular images depicting varying levels and locations of calcium deposits and other plaque within a vessel. The sample images may additionally be obtained from a large number of patients. The deep learning network may be trained using multiple annotated IVUS images. Each of the multiple annotated IVUS images include an annotation identifying the calcium.

As an example of one embodiment of training, each sample image selected for the training of a deep learning network may be assigned a variable $I_k$. Each image $I_k$ may be assigned two labels, $L_{k1}$ and $L_{k2}$, where $L_{k1}$ corresponds to the location of calcium deposits within the image and $L_{k2}$ corresponds to the location of other plaque within the image. The deep learning network may be trained batch-wise, using batches of tuples ($I_k$, [$L_{k1}$, $L_{k2}$]), where Ik is a possibly preprocessed ultrasound image, and $L_{k1}$ and $L_{k2}$ are corresponding labels. $L_{k1}$ and $L_{k2}$ may be in the form of a matrix of coordinates corresponding to the calcium or other plaque position respectively in an image. $L_{k1}$ and $L_{k2}$ may also be an overlay to be placed over the image Ik. In some embodiments, the labels for the locations of calcium deposits, $L_{k1}$, and the label for the locations of other plaque, $L_{k2}$, may be combined to form a single label, $L_k$. Training is then performed with the tuples ($I_k$, $L_k$), where $L_k$ includes the group of separate values describing the calcium and other plaque formations. For training the network, random batches of tuples ($I_k$, $L_k$) may be generated. The images may be forward-propagated through the deep learning network, creating a tentative label assignment $L_{k'}$ for each image. A loss function may be defined to measure the size of the error between $L_k$ and $L_{k'}$ for all labels in the batch. The error may then be back-propagated through the network and an optimizer is used to adjust the network's parameters in order to improve subsequent predictions. The training process may continue for a fixed number of iterations or until some convergence criterion is met. For example, the training process may continue until the error no longer improves for a specified number of iterations.

In one embodiment, the loss function used during the training of the deep learning network may measure whether the classification of calcium deposits and/or other plaque in an image is correct or incorrect. For example, given a finite set of labels that the deep learning network may be trained to identify, the network may assign probabilities for each of the classes. For instance, the network may assign a probability of 80% for one class and 1% for another classes. The loss function may then determine an error metric indicating how well the network predicts with high likelihood the correct label and low likelihood the incorrect labels. The loss function may include various other features or steps. For example, in an embodiment in which calcium deposit position is defined as a matrix of coordinates corresponding to locations within the received image, the loss function may include an error metric relating to the difference in coordinate values for each pixel or region between the predicted calcium location matrix and the actual calcium location matrix. One method for obtaining the labels $L_k$ is by having expert sonographers assign the label $L_k$ at the time of image acquisition (prospectively) or at the time of image review (retrospectively).

In some embodiments, additional values may be added to the ($I_k$, $L_k$) tuple corresponding to other desired outputs, including any of the outputs 730 shown in Fig. 7. Methods of training and generating these additional labels may be similar to the training of the labels $L_k$, $L_{k1}$, and/or $L_{k2}$ described previously.

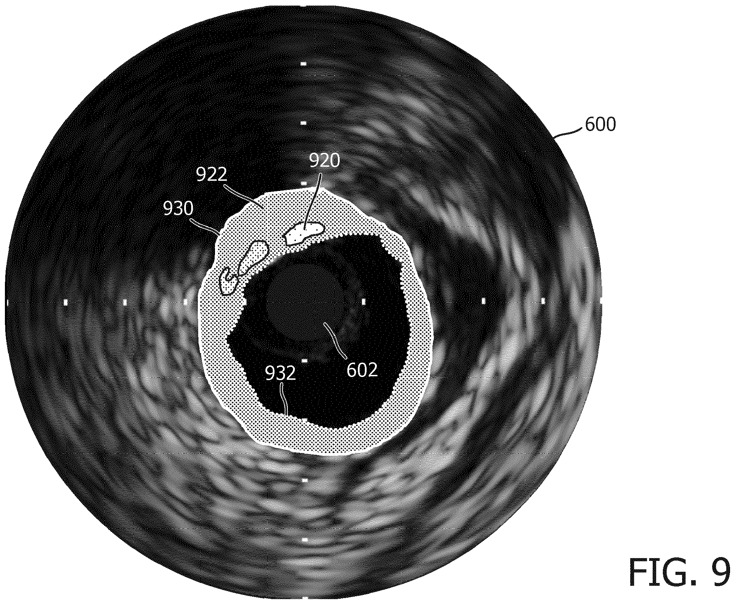

Specific aspects of Fig. 7 will be described in conjunction with Figs. 9-13. As shown in Fig. 7, the input images 710 may include any suitable number of intravascular ultrasound images. For example, the input images 710 may include only a single ultrasound image or may include multiple, as shown. The input images 710 can be an image stream of frames continuously acquired (e.g., in real time) by an imaging probe during a pullback procedure. The input images 710 can be transmitted from the ultrasound probe 102 (Fig. 1) to the analysis module 720 as the images are being acquired or after the images are acquired. In some embodiments, the ultrasound imaging probe 102 may transmit input images 710 to the analysis module 720 from a previous imaging procedure.

The analysis module 720 can include a preprocessor 722, which can include hardware (e.g., electrical circuit components) and/or software algorithms (e.g., executed by a processor). The preprocessor 722 may perform various functions to adjust, filter, or otherwise modify received input images 710 before transmitting the input images 710 to the deep learning network 724. For example, the preprocessor 722 may manipulate incoming images. The preprocessor 722 may perform various image processing steps for both training and prediction purposes. The preprocessor 722 may perform various tasks such as changing the contrast of the image, size of the image, resolution, orientation, geometry, cropping, spatial transformation, resizing, normalizing, histogram modification or various other procedures to assist deep learning network 724 to work more efficiently or accurately.

The deep learning network 724 may receive as an input the processed images 710 from the preprocessor 722. The deep learning network 724 can include hardware (e.g., electrical circuit components) and/or software algorithms (e.g., executed by a processor). The deep learning network 724 may then identify various parameters associated with the received ultrasound images 710.

The deep learning network 724 can include a convolutional neural network (CNN) in some embodiments. For example, the CNN can be or include a multi-class classification network, or an encoder-decoder type network. In some instances, the analysis module can implement any kind of classification process, such as a random forest algorithm, a classification tree approach, a convolutional neural network or any type of deep learning network. In some instances, the analysis module can implement a statistical model or random forest algorithm based on image or ultrasound backscatter derived parameters instead of a deep learning network.

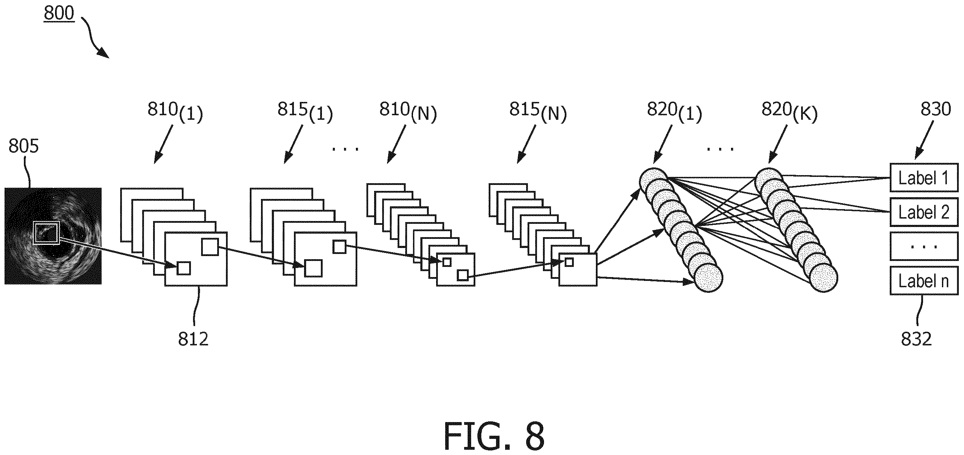

Fig. 8 is a schematic diagram of a convolutional neural network (CNN) configuration 800, according to aspects of the present disclosure. For example, the CNN configuration 800 can be implemented as the deep learning network 724 (Fig. 7). In an embodiment, the configuration 800 may perform a classification task. For example, a convolutional neural network (CNN) may provide classification labels for each pixel of an IVUS image or signal. The configuration 800 may be of any suitable type and may include any suitable type or number of layers including but not limited to convolutional layers, fully connected layers, flatten vectors, or any other techniques or implementations of artificial intelligence systems. The embodiments shown and/or described with reference to Fig. 8 can be scaled to include any suitable number of CNNs (e.g., about 2, 3 or more). The configuration 800 can be trained for identification of calcium deposits, other plaque, vessel borders, and/or lumen borders associated with received ultrasound images as described in greater detail below.

The CNN may include a set of N convolutional layers 810 where N is any positive integer, each layer followed by a pooling layer 815. The CNN may also include a set of K fully connected layers 820, where K may be any positive integer. In one embodiment, the fully connected layers 820 include at least two fully connected layers 820. The convolutional layers 810 are shown as 810(1) to 810(N). The pooling layers 815 are shown as 815(1) to 815(N). The fully connected layers 820 are shown as 820(1) to 820(K). Each convolutional layer 810 may include a set of filters 812 configured to extract features from an input 805 (e.g., ultrasound images or other additional data). The convolutional layers 810 may include convolutional kernels of different sizes and strides. The values N and K and the size of the filters 812 may vary depending on the embodiments. In some instances, the convolutional layers 810(1) to 810(N), the pooling layers 815(1) to 815(N), and the fully connected layers 820(1) to 820(K-1) may utilize a sigmoid, rectified non-linear (ReLU), leaky ReLU, softmax, or hyperbolic tangent activation function. The pooling layers 815 may include max pooling or average pooling techniques. The fully connected layers 820 may gradually shrink the high-dimensional output to a dimension of the prediction result (e.g., the classification output 830). Thus, the fully connected layers 820 may also be referred to as a classifier. In some embodiments, the fully convolutional layers 810 may additionally be referred to as perception or perceptive layers. The fully connected layers 820 may downsample and map received information to a finite number of classes 832. In an embodiment, the final fully connected layer 820(K) may be followed by a final classification layer such as softmax to transform the net activations in the final output layer to a series of values that can be interpreted as probabilities.

The classification output 830 may indicate a confidence score or probability for each of a plurality of classes 832, based on the input image 805. In that regard, the CNN 800 can be a multi-class classification network. In an exemplary embodiment, the plurality of classes 832 may include the location of calcium deposits within an intravascular image, the location of other plaque within the image, a vessel border in the image, or a lumen border in the image. The output 830 may indicate how likely various regions of the input image 805 belongs to or corresponds to a particular class 832. For example, a high confidence score for label 1 and lower confidence scores for the other labels indicates that the output label of the CNN 800 for a section of the input image 805 is label 1. For example, that section of the input image 805 may be classified as showing a calcium deposit. The confidence score may be of any suitable type. In one embodiment, the confidence score may be a probability (e.g., a decimal value between 0 and 1). In this way, the output of the CNN may be a likelihood value for each of the possible outputs for each section of the input image 805. In some embodiments, for a particular region of the input image, only outputs which exceed a predetermined threshold will be assigned to the region of the image. For example, if the likelihood value exceeds a minimum threshold, then the output label is assigned to that region of the image. If all output likelihood values are below the minimum threshold, no output classification will be selected or assigned to the region of the image, or a dedicated "unknown" label will be assigned. If the image is stored/archived, the sections of the image as well as the labels for each may be stored together with the image, for example in the DICOM header.

In an embodiment in which the deep learning network includes an encoder-decoder network, the network may include two paths. One path may be a contracting path, in which a large image, such as the image 805, may be convolved by several convolutional layers 810 such that the size of the image 805 changes in depth of the network. The image 805 may then be represented in a low dimensional space, or a flattened space. From this flattened space, an additional path may expand the flattened space to the original size of the image 805. In some embodiments, the encoder-decoder network implemented may also be referred to as a principal component analysis (PCA) method. In some embodiments, the encoder-decoder network may segment the image 805 into patches.

In some embodiments, multiple convolutional neural networks may be implemented to identify different characteristics of received ultrasound images. For example, one CNN may be trained to identify calcium plaque within a received ultrasound image, a separate CNN may be trained to identify other plaque, and additional CNN's may identify the vessel border and lumen border respectively. Any CNN may be trained to identify any one of these characteristics within a received ultrasound image, including just one, some, or all of these characteristics.

In some embodiments, aspects of the machine learning network may include a post-processing step. The post-processing step may combine outcomes of one or more classification or learning algorithms and/or apply a logic based on the size and spacing of pixels of an IVUS image corresponding to the presence of calcium or labelled as calcium pixels to provide an label, score, or metric. This post-processing step may be an additional algorithm to all other algorithms. This post-processing step may base a scoring of an IVUS image or individual pixels of an IVUS image both on quantitative analysis of the label clusters and published studies, literature, or empirical results from experts in the field. In some embodiments, this post-processing step may be an artificial algorithm itself.

Fig. 9 is a diagrammatic view of an intravascular ultrasound image 600 with an overlay, according to aspects of the present disclosure. Fig. 9 may represent the view of the ultrasound image 600 after the processor circuit 510 has completed implementation of the machine learning algorithm 700 (Fig. 7) and/or the convolutional neural network configuration (Fig. 8) to identify calcium deposits 920, other plaque 922, a vessel border 930, and a lumen border 932. Fig. 9 will be described with reference to Fig. 7.

As shown in Fig. 7, the deep learning network may be trained to identify calcium and plaque locations 731 within a received intravascular image 710. Similarly, as shown in Fig. 9, the system 100 may identify regions within the image 600 where calcium deposits 920 and other plaque 922 is present. Calcium deposits 920 and/or other plaque 922 may be observed at various locations within a vessel and may be part of other structures. As shown in Fig. 9, a large region of other plaque 922 is shown completely lining the vessel wall 930. As described, this other plaque 922 may include fibrous tissues, fibro-fatty tissues, lipids, complex carbohydrates, necrotic core, various dead blood cells or other tissues, or other materials which may build up over time. This build-up of other plaque 922 may restrict blood flow through the vessel. Depending on the composition of the plaque 922, the plaque 922 may exhibit different characteristics. For example, the plaque 922 may be hardened or flexible, may be more prone to movement or may be stationary, or may be prone to increasing or decreasing in size and/or severity. The presence of calcium deposits 920 within regions of other plaque 922 as shown in Fig. 9 may influence any of those characteristics and may alter the proper method of treatment.

The vessel border 930 and lumen border 932 are additionally identified by the deep learning network as shown in Fig. 9. In some embodiments, the vessel border 930 and lumen border 932 are identified by a user and received by the processor circuit. In some embodiments, the lumen border 932 may be the inner edge of the vessel wall. The lumen border may define the lumen. The machine learning algorithm 700 may be trained to identify the vessel border and lumen border 732. The processor circuit 510 may be configured to recognize the boundaries of the vessel within the ultrasound image as well as the boundaries of the lumen. By providing the user of the system 100 with a representation of the vessel border and lumen border, the user may more accurately determine the severity of an obstruction within the vessel and more accurately select a treatment method. Examples of border detection, image processing, image analysis, and/or pattern recognition include U.S. Pat. No. 6,200,268 entitled "VASCULAR PLAQUE CHARACTERIZATION" issued Mar. 13, 2001 with D. Geoffrey Vince, Barry D. Kuban and Anuja Nair as inventors, U.S. Pat. No. 6,381,350 entitled "INTRAVASCULAR ULTRASONIC ANALYSIS USING ACTIVE CONTOUR METHOD AND SYSTEM" issued Apr. 30, 2002 with Jon D. Klingensmith, D. Geoffrey Vince and Raj Shekhar as inventors, U.S. Pat. No. 7,074,188 entitled "SYSTEM AND METHOD OF CHARACTERIZING VASCULAR TISSUE" issued Jul. 11, 2006 with Anuja Nair, D. Geoffrey Vince, Jon D. Klingensmith and Barry D. Kuban as inventors, U.S. Pat. No. 7,175,597 entitled "NON-INVASIVE TISSUE CHARACTERIZATION SYSTEM AND METHOD" issued Feb. 13, 2007 with D. Geoffrey Vince, Anuja Nair and Jon D. Klingensmith as inventors, U.S. Pat. No. 7,215,802 entitled "SYSTEM AND METHOD FOR VASCULAR BORDER DETECTION" issued May 8, 2007 with Jon D. Klingensmith, Anuja Nair, Barry D. Kuban and D. Geoffrey Vince as inventors, U.S. Pat. No. 7,359,554 entitled "SYSTEM AND METHOD FOR IDENTIFYING A VASCULAR BORDER" issued Apr. 15, 2008 with Jon D. Klingensmith, D. Geoffrey Vince, Anuja Nair and Barry D. Kuban as inventors and U.S. Pat. No. 7,463,759 entitled "SYSTEM AND METHOD FOR VASCULAR BORDER DETECTION" issued Dec. 9, 2008 with Jon D. Klingensmith, Anuja Nair, Barry D. Kuban and D. Geoffrey Vince, as inventors, the teachings of which are hereby incorporated by reference herein in their entirety.

Fig. 10 is a diagrammatic view of an intravascular ultrasound image 1000 with an overlay including thickness and depth measurements, according to aspects of the present disclosure. Fig. 10 will be described with reference to Fig. 7. Fig. 10 depicts an additional intravascular image 1000 with regions of calcium deposits 1020 as well as other plaque build-up 1022 within a vessel. The processor circuit 510 (Fig. 5) may generate various metrics or measurements related to the location of the calcium deposits 1020, other plaque 1022, vessel border 1030, and lumen border 1032.

In some embodiments, referring again to Fig. 7, the outputs 730 of the machine learning algorithm 700 may be direct outputs based on the input intravascular images 710. For instance, the processor circuit 510 may be configured to implements the deep learning network 724 in such a way as to be able to generate any of the outputs 730 independently of one another. As an example, the deep learning network 724 may identify a frame score 738 associated with a particular received intravascular image 710 without also determining the calcium depth 733 or vice versa.

In some embodiments, various outputs 730 of the machine learning algorithm 700 may be determined based on other outputs 730. For example, in some embodiments, the calcium depth 733, calcium thickness 734, calcium arc 735, and calcium distribution 736 may be determined based on the identified calcium and plaque locations 731 and vessel and lumen borders 732. For instance, the identified calcium and plaque locations 731 and vessel border and lumen border 732 may be overlaid over a received intravascular image. The processor circuit 510 may then be configured to use the received image with the overlay identifying calcium deposits, other plaque formations, and vessel and lumen borders to determine calcium depth 733, calcium thickness 734, calcium arc 735, calcium distribution 736, and calcium length 737. The processor circuit 510 may be configured to employ various image processing techniques such as [list image processing techniques here]. In other embodiments, the processor circuit may employ a deep learning algorithm to generate the outputs 733-737. The deep learning algorithm used may be the same algorithm as the machine learning algorithm 700 described with reference to Fig. 7, the convolutional neural network 800 described with reference to Fig. 8, or may be a separate deep learning algorithm.

As shown in Fig. 10, locations within the image 1000 corresponding to calcium deposits 1020, other plaque 1022, the vessel border 1030, and the lumen border 1032 are identified. These features may be identified according to principles similar to those described with reference to the deep learning network algorithm of Fig. 7, the convolutional neural network of Fig. 8, or methods described with reference to Fig. 9.

Additionally shown in Fig. 10 is a scan line 1040. The processor circuit 510 may be configured to generate multiple scan lines like the scan line 1040 on the image 1000. The scan lines generated may or may not be displayed to a user. A scan line may be defined as a line from the center of the intravascular imaging device 602 within the image 1000 extending in a straight line to the outer border of the image 1000. A scan line may also correspond to an angle along which calcium depth and other measurements may be based. A scan line may be an angle from the centeroid of the vessel in which the plaque is present. In another embodiment, the image could be corrected for motion and aligned for vessel boundary, in which case the center of the image itself can be the annular center of the image. The processor circuit 510 may be configured to generate any number of scan lines within the image 1000. For example, the processor circuit 510 may generate a single scan line within the image 1000 or as many as 10,000 or more. In one embodiment, the number of scan lines generated within the image 1000 may be adjusted based on various characteristics of the image, the anatomy of the patient, or the ultrasound system 100. For example, the number of scan lines may be increased depending on the resolution of the image 1000, the measured overall diameter of the imaged vessel, the shape of the vessel, the distribution of calcium deposits and other plaque within the vessel, the processing capabilities of the processor circuit 510, storage limitations of the memory 564 (Fig. 5), or any other characteristics. The scan lines shown may further define sectors of the circular intravascular image 1000, as will be described in more detail hereafter.

As shown in Fig. 10, various measurements may be made along each scan line within the image 1000. For example, a calcium depth 1044 and a calcium thickness 1046 may be calculated along the scan line 1040 shown. The processor circuit 510 may be configured to use a deep learning network (e.g., the deep learning network 700 of Fig. 7) to determine the distance between the calcium and the lumen border (e.g., the calcium depth) and/or calcium thickness measurements. Calcium depth and calcium thickness measurements may be made anywhere in the vessel, or along any scan line of the image (e.g., extending from the catheter 602 center to the outer image border), where calcium or plaque is present. In some embodiments, the calcium depth may refer to the distance from the lumen border 1032 to a calcium deposit 1020. Calcium depth may be a distance between the calcium and the lumen border. In some embodiments, calcium thickness may refer to the distance from one end of a calcium deposit 2010 to the other end along a scan line. For example, the scan line 1040 of Fig. 10 passes through a calcium deposit 1023. The scan line 1040 illustrates a calcium depth 1044 and a calcium thickness 1046 corresponding to this calcium deposit 1023. The calcium depth 1044 is a measurement of the distance from the lumen border 1032 along the scan line 1040 to the inner edge of the calcium deposit 1023. The calcium thickness 1046 along the scan line is a measurement of the distance from the inner edge of the calcium deposit 1023 to the outer edge of the calcium deposit 1023 as shown. In some embodiments, the calcium depth may alternatively be referred to as a calcium thickness, distance to the lumen, lumen depth, closeness to the lumen, or other similar terms. In addition, in some embodiments, the calcium thickness may alternatively be referred to as a calcium depth.

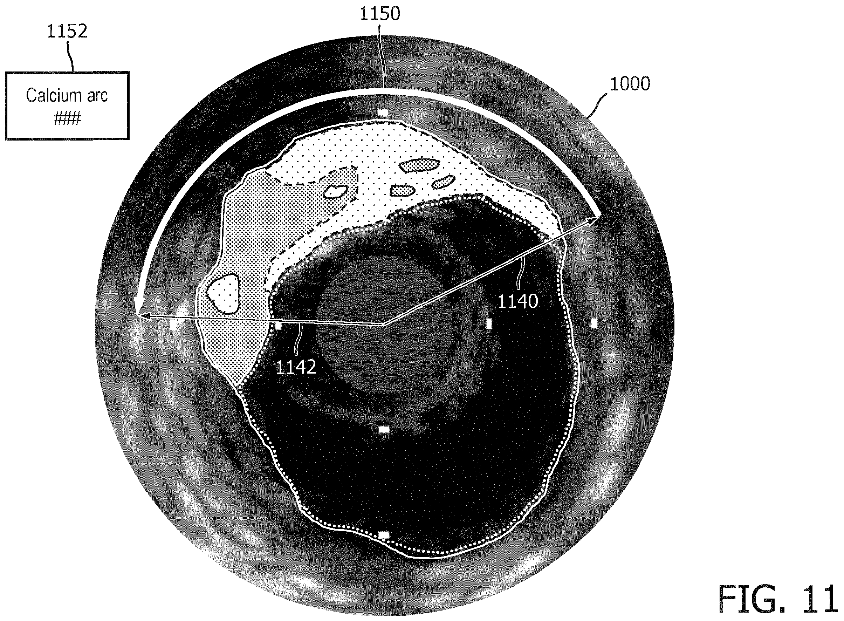

The calcium depth and thickness measurements shown may refer to measurements of distance which extend radially. A radial direction in Fig. 10 may refer to a direction extending from the center of the image 1000 to the outer edge of the image 1000. The distance between the calcium and lumen border may include a radial distance. In this way, each of the scan lines 1040, 1050, and 1060 extend outward radially. A circumferential direction may extend around the IVUS image 1000 as is shown by the arc 1150 of Fig. 11. In this way, the circumferential direction may be perpendicular or orthogonal to the radial direction. For example, a hypothetical curve of a constant radial distance may intersect each scan line of the image 1000 at a perpendicular angle. The radial and circumferential directions may be in the same plane. An additional longitudinal direction may refer to a direction along the imaged vessel. For example, the longitudinal direction may be perpendicular to both the radial and circumferential directions.

A calculation of calcium depth and thickness may be made along any suitable scan line within the image 1000. For example, an additional scan line 1050 is shown. The scan line 1050 passes through two regions of the same calcium deposit 1020. The measured calcium depth along the scan line 1050 may be 0 because the calcium 1020 is directly adjacent to the lumen. Stated differently, the inner surface of the calcium deposit 1020 defines the lumen border 1032 at a point along the scan line 1050. While the calcium depth 1044 along the scan line 1040 may be some positive value because a layer of other plaque 1022 separates the lumen border 1032 and the calcium deposit 1023, no other plaque separates the calcium deposit 1020 from the lumen border 1032, so no calcium depth will be given, or a calcium depth of 0 will be calculated along the scan line 1050. The calcium thickness 1056 may be calculated as the thickness of the first section of the calcium deposit 1020 along the scan line 1050. The calcium thickness along the scan line 1050 may be a measurement of the distance from the lumen border 1032, which is the same location as the inner edge of the calcium deposit 1020 along the scan line 1050, to the outer edge of the inner region of the calcium deposit 1020, as shown in Fig. 10.

As previously mentioned, the scan line 1050 extends across two regions of the same calcium deposit 1020, which will be referred to as the inner region being the region closest to the lumen border 1032, and outer region being the region closest to the vessel border 1030. Between these regions, a layer of other plaque 1022 is observed. The distance from the inner region of the calcium deposit 1020 to the outer region of the calcium deposit 1020 along the scan line 1050 may be referred to as a calcium depth 1058. In some embodiments in which a scan line passes through multiple regions of calcium and other plaque, distances of calcium deposits may be referred to as calcium thicknesses and distances of other plaque may be referred to as calcium depth. In such an embodiment, these measurements may be distinguished with various labels. For example, as shown in Fig. 10, the depth of 0 corresponding to the depth of the inner calcium region may be referred to as Calcium 1 Depth. Calcium thickness 1056 may be referred to as Calcium 2 Thickness. Calcium depth 1058 may be referred to as Calcium 2 Depth and calcium thickness 1059, illustrating the thickness of the outer region of the calcium deposit 1020, may be referred to as Calcium 2 Thickness and so on if additional regions of plaque and calcium were present along the scanline 1050.

As an additional example, a scan line 1060 is also shown. Because there are no regions of other plaque along the scan line 1060, the calcium depth, like the calcium depth along the scan line 1050, may be zero. In addition, the calcium thickness is equal to the distance from the lumen border 1032 to the vessel border 1030.

The processor circuit 510 (Fig. 5) may be configured to determine similar calcium depth and thickness measurements for each scan line created within the image 1000. After these calculations are made for each scan line, the processor circuit 510 may compare all calcium thickness values to determine a maximum and minimum calcium thickness. The processor circuit 510 may then identify which scan lines are associated with the maximum and minimum scan lines and identify those scan lines to a user of the system. For example, as shown in Fig. 10, the calcium thickness 1056 along scan line 1050 may be determined by the processor circuit 510 to be the minimum calcium thickness. The processor circuit may identify the scan line 1050 by overlaying a line corresponding to the scanline 1050 over the image on the display 132 (Fig.1). The line may be accompanied by a label including a symbol, graphical representation, or alphanumeric text identifying the scan line 1050 as the scan line associated with the minimum calcium depth. In some embodiments, the appearance of the scan line 1050 may be modified to convey that it is associated with the minimum thickness. For example, the scan line 1050 may be of any suitable pattern, color, weight, or other visual character to convey its designation as the scan line of minimum calcium thickness. Alternatively, processor circuit 510 may be configured to generate a graphical representation, similar to the arrow 1056 of Fig. 10 illustrating the calcium thickness along the scan line 1050. Such a graphical representation may be overlaid over the image in such as a way as to identify the location of the minimum calcium thickness and be of any suitable appearance to designate it as the minimum calcium thickness. In some embodiments, the scan line of minimum calcium thickness may be identified with an angle, or other measurement or positioning coordinates or values. Similarly, the calcium thickness 1066 shown in Fig. 10 may be a maximum calcium thickness measured by the system. The processor circuit 510 may identify the maximum calcium thickness using any of the methods discussed regarding the minimum calcium thickness, including by overlaying a scan line 1060, generating labels, symbols, graphical representations, alphanumeric texts, designating angles or coordinates, or by any other suitable method. In addition, the processor circuit 510 may be configured to compare all calcium depth measurements of each generated scan line. The processor circuit may then also identify minimum and maximum calcium depths within the image 1000. The processor circuit 1000 may identify minimum and maximum calcium depths and convey the identified minimum and maximum depths to the user by any of the methods used to describe the maximum and minimum calcium thickness values previously.

As shown in Fig. 10, the system described may be an intravascular ultrasound (IVUS) imaging system 100 (Fig. 1), including an IVUS imaging catheter 102 (Fig. 1) configured to obtain IVUS imaging data while positioned within a blood vessel of a patient. The blood vessel includes a wall formed of tissue comprising calcium 1020; and a lumen border 1032 defining a lumen. The processor circuit 510 (Fig. 5) of the IVUS imaging system 100 is configured for communication with the IVUS imaging catheter 102, wherein the processor circuit 510 is configured to receive the IVUS imaging data from the IVUS imaging catheter 102; identify the calcium 1020 based on the IVUS imaging data; and determine, based on the identification of the calcium, a distance between the calcium and the lumen border (e.g., a calcium depth 1044). As will be described with reference to Fig. 16, the processor circuit may then output a screen display 1600 (Fig. 16) to a display 132 (Fig. 1) in communication with the processor circuit 510 (Fig. 5), wherein the screen display 1600 includes an IVUS image 1640 generated based on the IVUS imaging data and a visual representation 1644 of the distance 1044. The system 100 (Fig. 1) may be an intraluminal imaging system.

The processor circuit may also be configured to calculate, based on the identification of the calcium, an individual numerical score representative of the calcium using a distance between the calcium deposit and the lumen border; a thickness of the calcium deposit; and angle spanned by the calcium deposit; and a length of the calcium deposit; and output a screen display to a display in communication with the processor circuit, wherein the screen display includes an IVUS image generated based on the IVUS imaging data and a visual representation of the individual numerical score. The individual numerical score may correspond to a scan line of the IVUS image, a sector of the IVUS image, an entirety of the IVUS image, or multiple IVUS images.

The processor circuit may be configured to calculate a distance between the calcium deposit and the lumen border, a thickness of the calcium deposit, an angle spanned by the calcium deposit, and a length of the calcium deposit. The distance, the thickness, the angle, and the length may be weighted based on the location of the calcium within the wall. Any of the parameters described herein, for example, distances between the calcium deposit and the lumen border, a thickness of the calcium deposit, an angle spanned by the calcium deposit, and a length of the calcium deposit may all be considered in the determination of a volume measurement. For example, the processor circuit may use any of these measurements to determine a volume measurement of a particular calcium deposit.

Fig. 11 is a diagrammatic view of an intravascular ultrasound image with an overlay including a calcium arc measurement 1152, according to aspects of the present disclosure. Fig. 11 will be described with reference to Fig. 7. As shown in Fig. 7, an additional output 730 of the machine learning algorithm 700 may be a calcium arc 735. As previously mentioned, the calcium arc 735 may be a direct output of the machine learning algorithm 700 and independent of any other outputs 730. However, in some embodiments, the calcium arc 735 may be determined by the processor circuit 510 (Fig. 5) based on various outputs 730, such as the calcium and other plaque locations 731.

Referring again to Fig. 11, a calcium arc measurement 1152 is displayed adjacent to the intravascular image 1000. Additionally, a calcium arc 1150 is illustrated within the image along with two scan lines 1140 and 1142. In some embodiments, the processor circuit 510 (Fig. 5) may determine whether and to what extent calcium deposits are present along each scan line within the image 1000. The angle of each scan line at which calcium deposits were identified may be stored together. These scan lines which identify calcium deposits may together define a calcium arc. For example, the scan lines 1140 and 1142 shown in Fig. 11 may identify first and last scan lines within the image 1000 identifying calcium deposits. Specifically, each scan line between the scan lines 1140 and 1142 may indicate the presence of calcium deposits. And, as shown in Fig. 11, such is the case. For any scan line extending from the center of the imaging assembly 602 to the outer border of the image in a straight line between the scan lines 1140 and 1142 as shown by the calcium arc 1150, some amount of calcium is present within the vessel. The identification of the calcium arc 1150 may assist a physician in quickly and easily identify all locations within the vessel of calcium deposits. This information may be conveyed to the user by displaying the calcium 1150 overlaid on the image 1000 or positioned around the image 1000. Alternatively, angles corresponding to the scan lines 1140 and 1142 may be displayed as the calcium arc measurement 1152. Alternatively, the calcium arc measurement 1152 may be the difference between the angles of the scan lines 1140 and 1142 or the angle of the scan line 1140 to the scan line 1142. The calcium arc measurements 1152 may be positioned adjacent to the image 1000, overlaid over the image 1000 or displayed in any other way.

In some situations, multiple calcium arcs and associated arc measurements may be present within an image 1000. For example, if one or more scan lines between the scan lines 1140 and 1142 did not indicate the presence of calcium deposits, two calcium arcs may be present and conveyed to the user of the system 100. In this regard, the calcium arc 1150 may not be a continuous arc or the calcium deposit identified by the arc 1150 may not be a continuous piece of calcium. Rather, it may be layered or broken up. There may be multiple calcium deposits separated by scan lines indicating no calcium presence.

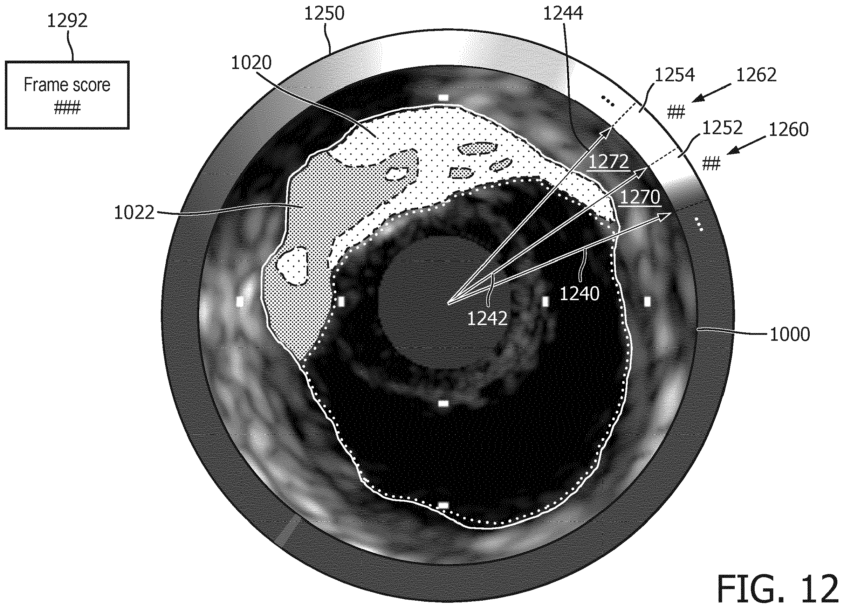

Fig. 12 is a diagrammatic view of an intravascular ultrasound image 1000 with an overlay including a calcium composition ring 1250, according to aspects of the present disclosure. Fig. 12 illustrates the intravascular image 1000 with the calcium deposits 1020 and other plaque 1022 as well as three scan lines 1240, 1242, and 1244 defining two sectors 1270 and 1272. Each sector 1270 and 1272 may correspond to a scan line score 1260. The calcium composition ring 1250 shown in Fig. 12 may be referred to as a visual representation of calcium location. Fig. 12 will be described with reference to Fig. 7. As shown in Fig. 7, additional outputs 730 of the machine learning algorithm 700 may include scan line scores 739 and a visual representation of calcium location 740. The scan line scores 739 and visual representation of calcium location 740 may be direct outputs of the machine learning algorithm 700 and independent of any other outputs 730. However, in some embodiments, the scan lines scores 739 and visual representation of calcium location 740 may be determined by the processor circuit 510 (Fig. 5) based on various outputs 730, such as the calcium and other plaque locations 731, the vessel border and lumen border 732, calcium depth 733, calcium thickness 734, calcium arc 735, calcium distribution 736, and/or calcium length 737.

As previously described, the processor circuit 510 (Fig. 5) may generate multiple scan lines extending from the center of the imaging assembly to the outer borders of the intravascular image 1000. The multiple scan lines generated may divide the circular intravascular image into an equal number of sectors. For example, as shown, the scan lines 1240 and 1242 may define a sector 1270 of the image 1000. Similarly, the scan lines 1242 and 1244 may define a sector 1272 of the image 1000. It is understood that additional scan lines may be generated in any direction around the image 1000 such that the entire image 1000 is divided into similarly sized and shaped sectors.

In some embodiments, the processor circuit 510 may be configured to analyze each sector of the image 1000 to determine whether calcium deposits 1020 or other plaque 1022 are found within each sector. The processor circuit 510 may additionally determine to what extent calcium deposits exist within the sector. For example, measurements corresponding to the calcium depth and calcium thickness may be used to determine the extent of calcification shown within the sector. The processor circuit 510 may additionally use measurements of calcium length 737 (Fig. 7) and calcium distribution 736 (Fig. 7) to determine the extent of calcium presence in a sector.

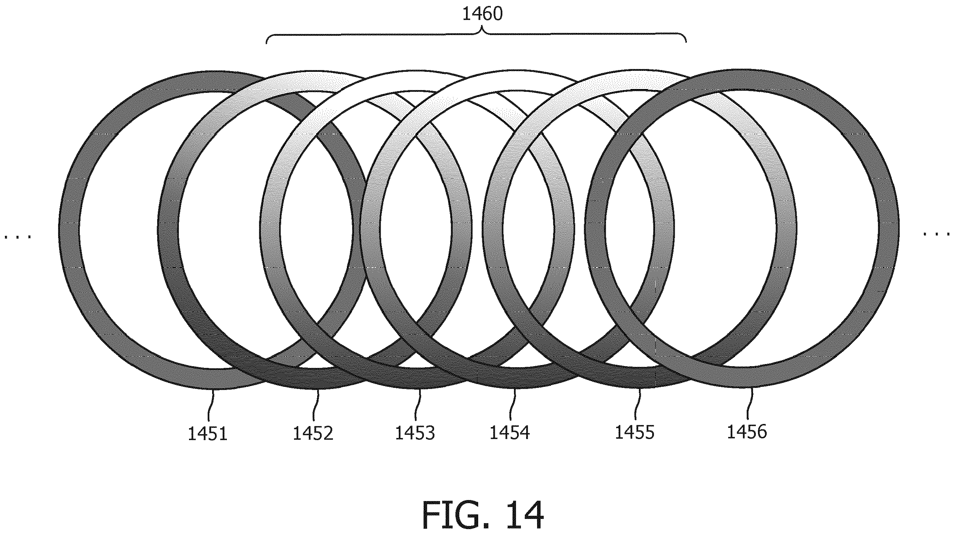

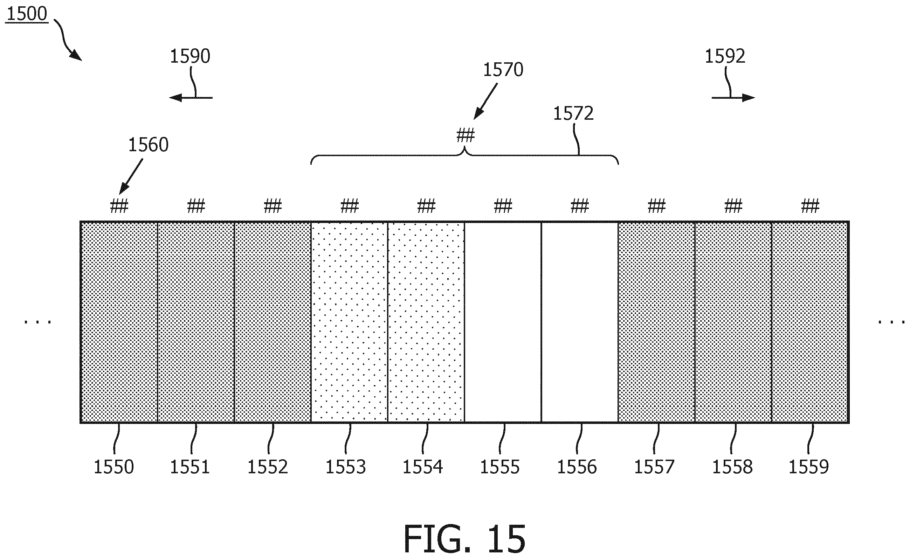

Calcium length 737 (Fig. 7) may be defined as the distance lengthwise along the imaged vessel at which calcium is found. Calcium length may be determined by receiving multiple intravascular images which were obtained by the intravascular imaging device sequentially or consecutively. For example, as will be discussed in greater detail with reference to Fig. 14 and Fig. 15, multiple intravascular images may be received by the system 100. The processor circuit may perform analysis on each received image according to methods described previously with reference to Figs. 7-12 to determine the location of calcium and plaque within each received image. In this way, the size of a calcium deposit may be defined by calcium thickness measurements (e.g., a radial measurement), calcium arc measurements (e.g., an angular measurement), and calcium length measurements (e.g., a longitudinal measurement) to quantify a the size and shape of a calcium deposit in three dimensions. As described, each received intravascular image may be divided into sectors with multiple sectors. As a result, calcium length may be determined for entire frames or for an entire calcium deposit, or calcium length may be determined for individual sectors across multiple consecutive or neighboring intravascular images. To determine the extent of calcification of one sector of an image, sectors of the same angles of neighboring images along the vessel may be analyzed for calcium content as well to determine the calcium length corresponding with the sector analyzed.

Calcium distribution 736 (Fig. 7) may refer to the size and position of calcium deposits within a vessel in relation to other plaque as well as stability of the calcium deposits. Calcium distribution 736 may be quantified according to various methods and may provide the physician with an accurate view of the location and amount of calcium relative to other material within an obstruction.

In some embodiments, calcium distribution 736 may be quantified in whole or in part by a calcium density measurement. For example, a calcium distribution measurement may include a linear density measurement, an area density measurement, or a volume density measurement. The processor circuit 510 may be configured to use a deep learning network (e.g., the deep learning network 700 of Fig. 7) to determine the density of the calcium relative to the other tissue. The processor circuit 510 may be configured to use a deep learning network (e.g., the deep learning network 700 of Fig. 7) to determine the distance between the calcium deposit and the lumen border, the thickness of the calcium deposit, the angle spanned by the calcium deposit, and the length of the calcium deposit. The distance between the calcium deposit and the lumen border may extend radially. The thickness of the calcium deposit may extend radially. The angle spanned by the calcium deposit may extend circumferentially. The length of the calcium deposit may extend longitudinally.

A linear calcium density measurement may describe the amount of calcium relative to other material along a single scan line. For example, a linear calcium density measurement may be determined based on the number of pixels along a particular scan line showing calcium. A linear calcium density may be calculated as a percentage of pixels along a scan line. The number of pixels along the scan line showing calcium may be divided by the total number of pixels along the scan line to yield a linear density value. In some embodiments, other units of measurement of length may also be used by the processor circuit to make a linear density calculation. A linear density measurement may also be referred to as a one-dimensional density measurement. In some embodiments, the processor circuit may determine a linear density measurement along each scan line within the IVUS image. In some embodiments, the processor may make these calculations in response to a user input. A user of the system may create various custom lines within an IVUS image, which may be scan lines extending from the center of the IVUS device to the vessel wall or may be lines extending in any direction within the image. The processor circuit may determine a linear density value corresponding to the user-created line in response to a user input.

An area calcium density measurement may describe the amount of calcium relative to other material within a sector, or other two-dimensional and/or cross-sectional region of an IVUS image or the vessel imaged. For example, an area calcium density measurement may be determined based on the number of pixels within a particular sector of an IVUS image showing calcium. An area calcium density may be calculated as a percentage of pixels within the sector. The number of pixels within the sector showing calcium may be divided by the total number of pixels within the sector to yield an area density value. In some embodiments, other units of measurement of area may also be used by the processor circuit to make an area density calculation. An area density measurement may also be referred to as a surface density measurement or a two-dimensional density measurement. The user of the system may define sectors within an IVUS image or other custom regions or shapes within the IVUS image. The processor circuit may then determine an area density measurement for the sector or other custom shape defined by the user in response to a user input. For example, the user may identify the outline of an obstruction within a vessel. The processor circuit may determine an area calcium density measurement corresponding to the identified region. In some embodiments, the processor circuit may user methods previously described to automatically identify a region within an IVUS image corresponding to an obstruction and calculate an area calcium density for the region. In some embodiments, the processor circuit may determine an area calcium density measurement for an entire IVUS image.

A volume calcium density measurement may describe the amount of calcium relative to other material within a three-dimensional region of the vessel. Such a region may be observed in multiple adjacent IVUS images. A volume calcium density measurement may be determined based on the number of pixels within a particular three-dimensional region of multiple IVUS images showing calcium. A volume calcium density may be calculated as a percentage of pixels within the three-dimensional region. The number of pixels within the region showing calcium may be divided by the total number of pixels within the three-dimensional region to yield a volume density value. In some embodiments, other units of measurement of volume may also be used by the processor circuit to make a volume density calculation. A volume density measurement may also be referred to as a three-dimensional density measurement. The user of the system may define regions within an IVUS image or multiple IVUS images. The processor circuit may then determine a volume density measurement for the region defined by the user in response to a user input. For example, the user may identify the outline or surface of an obstruction within a vessel in three-dimensions. This may be accomplished by identifying the outline of an obstruction in multiple adjacent IVUS images. The processor circuit may determine a volume calcium density measurement corresponding to the identified region. In some embodiments, the processor circuit may automatically identify a three-dimensional region within a vessel by automatically identifying an outline of an obstruction within multiple adjacent IVUS images using methods previously described. The processor circuit may then automatically calculate the volume calcium density for that region. As described, the processor circuit may be configured to determine the density for a scan line of the IVUS image (e.g., a linear calcium density), a sector of the IVUS image (e.g., an area calcium density), an entirety of the IVUS image (e.g., an area calcium density), and/or multiple IVUS images (e.g., a volume calcium density).

In some embodiments, measurements of calcium distribution may be quantified as a value within a scale, such as a value between 0 and 5, 0 and 10, 0 and 100 or any other suitable scale. For example, for a calcium distribution scale of 0 to 5, a sector containing a block of solid calcium with no other plaque may result in a calcium distribution factor of 5. Similarly, a sector showing no calcium may result in a calcium distribution factor of 0. Sectors with varying amounts or mixtures of calcium and other plaque may result in other corresponding distribution factors between 0 and 5. In some embodiments, the calcium distribution factor may also be referred as a confluency factor. Calcium distribution may also be represented and/or quantified as an area in units of mm2, or as a ratio or percentage of an overall plaque area as compared to a total vessel cross-sectional area. In some cases, a calcium distribution score may be, for example, a classification of disease or constriction severity. In some cases, different methods of representing or quantifying the calcium distribution may provide a user with varying levels of specificity. The particular method of quantification (i.e., a distribution score, cross-sectional area in mm2, a ratio or percentage, etc.) may be a user adjustable setting and may be based on the user's skill, experience, and/or level of expertise. Any of these quantifications of a calcium distribution may be displayed to a user either simultaneously or separately.

In some embodiments, a calcium distribution may not be quantified. However, the calcium may be classified based on quantitative and qualitative criteria. The quantification can be derived from various texture-based and/or image-parameters and also by analysis of backscattered imaging data. The scoring or quantification could also be linked to a particular treatment strategy on its own or when combined with other parameters like the arc, depth, area, volume, etc.

The processor circuit 510 (Fig. 5) may determine a scan line score based on any or all of calcium depth, calcium thickness, calcium length, and calcium distribution measurements from the corresponding sector. In some embodiments, the scan line score may also be referred to as a sector score.

As an example shown in Fig. 12, a scan line score or sector score 1260 corresponds to the sector 1270. The sector 1270 may be defined by the scan lines 1240 and 1242. A section of calcium is observed within the sector 1270. As a result, a calcium thickness measurement, calcium depth measurement, calcium length measurement, and calcium distribution factor may each contribute to the calculation of the sector score 1260. The processor circuit 510 (Fig. 5) may employ any suitable methods to determine the sector score 1260. In some embodiments, the sector score may be defined as a function of any or all of the calcium thickness, calcium depth, calcium length, and/or calcium distribution. For example, a function $f$ may be defined dependent on $Ca_{thickness}$ (calcium thickness), $Ca_{depth}$ (calcium depth), $Ca_{length}$ (calcium length), and $Ca_{distribution}$ (calcium distribution). As an example, the function f may be defined as a weighted sum such as:

$$f\left(Ca_{thickness}, Ca_{depth}, Ca_{length}, Ca_{distribution}\right)$$

$$= w_1\, Ca_{thickness} + w_2\, Ca_{depth} + w_3\, Ca_{length} + w_4\, Ca_{distribution}$$

wherein $w_1$ is a constant value weighting calcium thickness, $w_2$ is a constant value weighting calcium depth, $w_3$ is a constant value weighting calcium length, and $w_4$ is a constant value weighting calcium distribution. In some embodiments, the values $w_1$, $w_2$, $w_3$, and $w_4$ may alternatively be functions dependent on any suitable factors such as a distance of calcium or plaque from a vessel border and/or lumen border, overall diameter of an imaged vessel or lumen, calcium arc, or any other suitable factors. In some embodiments, the function used by the processor circuit 510 (Fig. 5) may include a weighted average, weighted integral, or any other suitable function.

In some embodiments, the processor circuit 510 may employ a deep learning network to determine a sector score. For example, the determination of a sector score may be a part of the machine learning algorithm 700 described with reference to Fig. 7 and/or the convolutional neural network 800 described with reference to Fig. 8. In other embodiments, a separate deep learning network may be implemented receiving calcium thickness, calcium depth, calcium length, and/or calcium distribution measurements from within the selected sector as inputs to produce a sector score.

A sector score may be quantified in any suitable way. For example, in some embodiments, a sector score may be quantified as a percentage. In other embodiments, a sector score may be quantified as a value within a scale, such as a value between 0 and 5, 0 and 10, 0 and 100 or any other suitable scale. For example, in one embodiment, a sector score scale may be defined as values between 0 and 3 in which allowable scores are limited to integers. For example, a sector of the image 1000 containing no calcium may be assigned a sector score of 0. While a sector containing a level of calcium at or near a maximum and/or otherwise determined to be severe according to the sector score function or deep learning network may be assigned a sector score of 3. A sector associated with a less severe presence of calcium may be assigned a sector score of 2 and one associate with an even less severe presence of calcium may be assigned a sector score of 1.

Similar to the scan line or sector score 1260, the scan line score or sector score 1262 may be determined based on attributes of the sector 1272. For example, like the sector score 1260, the sector score 1262 may be a function of the calcium depth, calcium thickness, calcium length, and/or calcium distribution observed within or corresponding to the sector 1272. Any similar sector around the image 1000 may similarly be associated with a sector score. The sector scores determined by the processor circuit 510 may then be used to generate the calcium composition ring 1250 shown.

In some embodiments, the calcium composition ring 1250 may be of a circular shape with an outer and inner diameter. The inner diameter of the calcium composition ring 1250 may be equal to the diameter of the intravascular image received. In some embodiments, the calcium composition ring may be centered around the intravascular imaging device shown in the intravascular image 1000. The calcium composition ring 1250 may be of any suitable shape or appearance. In some embodiments, the color of the calcium composition ring 1250 may be determined by attributes of the intravascular image 1000. For example, the color of the calcium composition ring 1250 may be determined by attributes of individual sectors within the image 1000. In some embodiments, the color of the calcium composition ring 1250 may be determined by the sector scores associated with the sectors of the image 1000. In some embodiments, the calcium composition ring 1250 may be a different shape. For example, rather than a ring around the IVUS image 1000, a plotted line may be positioned around the image 1000 illustrating, by the position of a line, the extent of calcium along a particular scan line. In still other embodiments, a bar graph may be placed around the image where the height of individual bars may correspond to an increased presence of calcium along an associate scan line or sector.

For example, if the sector scores of the image 1000 are defined as a value between 0 and 5, in which a score of 0 indicates no presence of calcium within the associate sector and 5 indicates a region of solid calcium within a sector, one color may be assigned to the sector score 0 and a different color may be assigned to the sector score 5. A gradient between the colors may be defined such that scores between 0 and 5 may produce a different color along the gradient. In the example shown in Fig. 12, a sector score of 5, indicating a region of solid calcium, may be assigned the color white. A sector score of 0 may be assigned a dark gray color. Because the region of calcium deposit 1020 within the sector 1272 is a solid block of calcium, the sector score 1262 may be 5, and, as shown, the region 1254 of the ring 1250 may be white. Alternatively, the region 1270 does not correspond to a solid block of calcium deposit 1020. Instead, part of the sector 1270 includes regions of solid calcium deposit 1020, but other regions of the sector do contain neither calcium nor plaque deposits. As a result, the sector score 1260 may be some value between 0 and 5, such as 3. The color of the region 1252 of the ring 1250 may not be white. In some embodiments, the region 1252 could be a solid non-white color, such as a lighter gray. However, in some embodiments, as shown in Fig. 12, the region 1252 may include a gradient, in which parts of the region 1252 are white corresponding to parts of the sector 1270 of solid calcium and parts of the region 1252 may be other colors corresponding to parts of the sector 1270 of other calcium compositions, including no calcium presence. Other regions of the ring 1250 are shown to correspond to the calcium presence and/or distribution within the image 1000.

It is noted that the scan line or sector scores of the image 1000 may be determined in any suitable manner. For example, in some embodiments, as explained previously, a score may be dependent on the calcium depth or thickness. In such an example, a score of 5 may refer to a region of solid calcium that is also very thick. A region of solid calcium of lesser thickness may result in a score of less than 5. As previously discussed, any of calcium depth, calcium thickness, calcium length, and/or calcium distribution may influence the sector scores.

Scan line or sector scores may also be determined based on information related to any of the scan lines of the image 1000. For example, rather than analyzing data within a sector, the processor circuit 510 may analyzed data along an individual scan line. This data may include any of the attributes described with reference to sector analysis previously described. For example, calcium depth, thickness, length, and/or distribution may influence the determination of a scan line score.

In addition to the calculation of scan line scores or sector scores, the processor circuit 510 (Fig. 5) may also determine an overall frame score 1292 associated with the entire image 1000. The overall frame score 1292 may be a simplified measure of the calcium presence and distribution within an individual intravascular image 1000. The processor circuit may be configured to determine an individual numerical score (e.g., a frame score, scan line score, sector score, etc.) representative of the calcium, wherein the individual numerical scores is based on the distance between the calcium and the lumen border (e.g., the calcium depth) and/or the density of the calcium relative to the other tissue (e.g., the calcium distribution). Like the scan line scores or sector scores previously described, the overall frame score 1292 may be dependent on the calcium thickness, depth, length, and/or distribution. However, because within one particular intravascular image 1000 there may be many measurements of calcium thickness, depth, length, and distribution made (e.g., associated with each scan line and/or sector of the image 1000), the overall frame score 1292 may combine or simplify many of these measurements. For example, in the determination of the overall frame score 1292, the processor circuit may calculate an average, median, maximum, minimum, range, standard deviation, or any other statistical calculation of any or all of the calcium depth, thickness, length, or distribution. In addition, the overall frame score 1292 may be dependent on the calcium arc. Referring back to Fig. 11, the calcium arc may define the angles at which calcium is present. The overall frame score 1292 may additionally be determined based on any or all of the scan line scores or sector scores of the image 1000.

In some embodiments, the overall frame score 1292 may be of a similar format or range as the scan line or sector scores. For example, if the scan line or sector scores are defined as values between 0 and 5 with 0 indicating no calcium presence and 5 indicating maximum calcium presence, the overall frame score 1292 may be defined in the same way. In other embodiments, the format or range of the overall frame score 1292 may differ from the scan line or sector scores. Similar to the scan line scores or sector scores, the overall frame score 1292 may be of any suitable format.

The overall frame score 1292 may be displayed to the user in any suitable way. For example, the overall frame score 1292 may be positioned adjacent to the image 1000 as shown in Fig. 12. Alternatively, the overall frame score 1292 may be overlaid over the image 1000, may be positioned along or within an in-line digital or image longitudinal display (ILD) or overlaid on an angiography image adjacent to the location along the vessel at which the intravascular image 1000 was acquired. The overall frame score 1292 may be conveyed to the user by any other method, including through various colors, patterns, shapes, graphical representations, sounds, haptic feedback, or any other method.

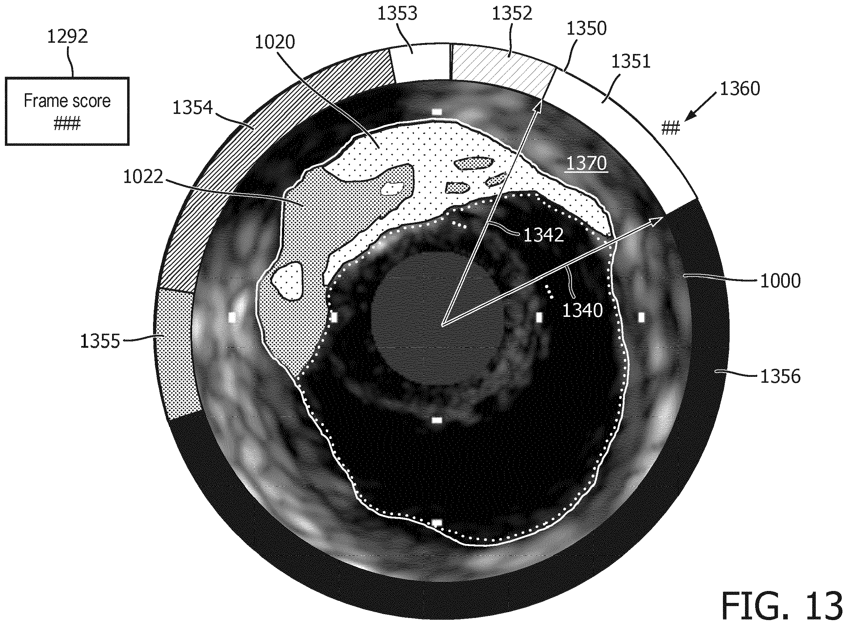

As shown in Fig. 12, the system described may be an intravascular ultrasound (IVUS) imaging system 100 (Fig. 1), including an IVUS imaging catheter 102 (Fig. 1) configured to obtain IVUS imaging data while positioned within a blood vessel of a patient. The blood vessel includes a wall formed of tissue comprising calcium 1020; and a lumen border 1032 defining a lumen. The processor circuit 510 (Fig. 5) of the IVUS imaging system 100 is configured for communication with the IVUS imaging catheter 102, wherein the processor circuit 510 is configured to receive the IVUS imaging data from the IVUS imaging catheter 102; identify the calcium 1020 based on the IVUS imaging data; and determine, based on the identification of the calcium, a density between the calcium and the lumen border (e.g., a calcium distribution 736). As will be described with reference to Fig. 16, the processor circuit may then output a screen display 1600 (Fig. 16) to a display 132 (Fig. 1) in communication with the processor circuit 510 (Fig. 5), wherein the screen display 1600 includes an IVUS image 1640 generated based on the IVUS imaging data and a visual representation of the density (e.g., the frame scores 1692, 1660, 1670, distribution ring 1650, regions 1662, 1664, 1666, 1668, 1612, 1614, 1616, or 1618 of Fig. 16, scores 1260, 1262, or 1292 of Fig. 12, regions 1351, 1352, 1353, 1354, 1355, or 1356, or score 1360 of Fig. 13, the rings or score 1460 of Fig. 14, or the scores 1560, 1570 or frames 1550 through 1559 of Fig. 15). The system 100 (Fig. 1) may be an intraluminal imaging system. Fig. 13 is a diagrammatic view of an intravascular ultrasound image 1000 with an overlay including a calcium composition ring 1350, according to aspects of the present disclosure. Fig. 13 illustrates the intravascular image 1000 with the calcium deposits 1020 and other plaque 1022 as well as two scan lines 1340 and 1342 defining a sector 1370. The sector 1370 may correspond to a region of the calcium composition ring 1350 and a sector score 1360.

Similar to the scan lines 1240, 1242, and 1244 of Fig. 12, the scan line 1340 and 1342 may extend from the center of the intravascular imaging device shown within the image 1000 to the outer border of the image 1000. Additional scan lines may be generated within the image 1000 in Fig. 13.

Like the methods described with reference to Fig. 12, the processor circuit 510 may generate multiple scan lines within the image 1000. Each scan line may correspond to various calcium measurements including one or more calcium depth measurements, one or more calcium thickness measurements, one or more calcium length measurements, and/or one or more calcium distribution measurements. These measurements may be calculated for each scan line generated within the image 1000. As described previously, these measurements may be used to determine various scan line scores or sector scores.

In the embodiment shown in Fig. 13, the processor circuit 510 (Fig. 5) may be configured to identify regions of the image 1000 of the same or similar scan line or sector scores and display these regions to the user. For example, as shown in Fig. 13, the processor circuit 510 may determine that all scan lines between the scan lines 1340 and 1342 within the image 1000 correspond to the same or similar scan line or sector scores. For example, the sector score of each scan line within this region may be 5 on a scale of 0 to 5 indicating that each scan line between the scan lines 1340 and 1342 correspond to a region of calcium that is a solid block of calcium without any presence of other plaque. As a result, the scan lines 1340 and 1342 may define a sector 1370. The sector 1370 may be associated with a region 1351 of the calcium composition ring 1350. The color and pattern of the region 1351 of the calcium composition ring 1350 may quickly convey to the user that throughout the sector 1370, from the scan line 1340 to the scan line 1342, calcium is present and it is a solid block of calcium, without any presence of other plaque.

The processor circuit 510 may perform similar measurements such as scan line score or sector score assignments throughout the image 1000 and may group other regions of the image accordingly. For example, a region 1352 of the calcium distribution ring 1350 may indicate to the user that within the corresponding sector of the image 1000, calcium 1020 is present with a relatively small amount of other plaque 1022. A region 1353 may indicate, like the region 1351, that calcium is present and it is a solid block of calcium, without any presence of other plaque. A region 1354 may indicate that calcium 1020 is present with relatively large regions of other plaque 1022. A region 1355 may indicate that no calcium 1020 is present within the corresponding sector, but that a solid block of other plaque 1355 is present. Finally, a region 1356 may indicate that no calcium 1020 or other plaque 1022 is present in the corresponding sector.

In the embodiment shown in Fig. 13, various colors and patterns may be used to convey attributes of sectors of the image 1000 to the user. For example, the color of regions of the calcium distribution ring 1350 may correspond to the amount of calcium within a sector, including measurements such as the calcium depth and thickness. The pattern of the regions of the calcium distribution ring 1350 may correspond to the distribution of the calcium indicating to the user whether the calcium is a solid block or is part of a distribution of other plaque and/or may indicate the proportion of calcium and other plaque. Additional visual attributes of regions of the calcium ring 1350 may convey other measurements to the user. For example, other attributes may be used to convey the calcium length. In some embodiments, the user of the system 100 may modify or customize which colors, patterns, or other visual attributes are associated with different measurements of the image 1000.

In some embodiments, the processor circuit 510 may be configured to group scan lines of the same scan line score into the same region of the calcium distribution ring 1350. In other embodiments, the processor circuit 510 may be configured to group scan lines of similar scan line scores into the same region of the calcium distribution ring 1350. For example, the processor circuit or a user of the system 100 may define a maximum difference in scan line score value. All scan lines which fall within this maximum range of scan line scores and which are positioned next to each other, or consecutively around the IVUS image 1000, may be grouped into the same region of the image 1000, such as the region 1351. In some embodiments, scan lines may be grouped into the same regions of the calcium distribution ring 1350 according to any other particular attributes of the scan lines or image 1000. For example, consecutive scan lines each corresponding to a region of solid calcium, despite scan line scores, calcium thickness, or calcium depth, may be grouped into the same region within the calcium distribution ring 1350.

In some embodiments, regions of the ring 1350 may be defined by specific measurements associated with corresponding sectors or scan lines of the image 1000. For example, regions of the ring 1350 may be defined or grouped according to calcium depth, calcium thickness, calcium length, or calcium distribution. In some embodiments, the processor circuit 510 may be configured to generate a calcium distribution ring 1350 related to only one of calcium depth, calcium thickness, calcium length, or calcium distribution in response to a user of the system 100 selecting the parameter. The calcium distribution ring 1350 may be generated such that it contains colors and patterns arranged in a gradient pattern, such as the ring 1250 shown in Fig. 12 or regions of varying colors and/or patterns may be arranged discreetly, as shown in Fig. 13.

Fig. 14 is a diagrammatic view of multiple calcium composition rings, according to aspects of the present disclosure. Fig. 14 includes one example of a method of providing a user of the system 100 a simple and efficient method of illustrating calcium distribution in both an angular direction around a vessel and a longitudinal direction along the vessel. Fig. 14 includes several calcium composition rings, including rings 1451, 1452, 1453, 1454, 1455, and 1456. The calcium composition rings shown in Fig. 14 may be similar to the calcium composition ring 1250 described with reference to Fig. 12 and/or the calcium composition ring 1350 described with reference to Fig. 13. For example, the calcium composition rings shown in Fig. 14 may illustrate the regions within associated intravascular images at which calcium deposits are present and what extent calcium is present at those locations. For example, the calcium composition ring 1451 is of a uniform, dark gray color. In some embodiments, this may represent a calcium composition ring associated with an intravascular image showing no presence of calcium deposits. Adjacent to the ring 1451, a ring 1452 is shown. The ring 1452 may be associated with an intravascular image which was acquired directly before or after the image associated with the image 1451. In this way, the ring 1452 may correspond to a location within the imaged vessel directly proximal or distal to the location of the ring 1451. As a result, the calcium composition rings shown in Fig. 14 may illustrate locations within the imaged vessel extending longitudinally.

The ring 1452 may correspond to an intravascular image showing a calcium deposit at the upper region of the image, as shown by the whitened coloration of the ring 1452 in that region. Similarly, the ring 1453 may correspond to an intravascular image showing a calcium deposit at the upper region of the image, as shown by the whitened coloration of the ring 1453 in that region. Because the whitened region of the ring 1453 is larger than the whitened region of the ring 1452, the user of the system 100 may quickly deduce that the location of the vessel associated with the ring 1453 contains a higher calcium content than the ring 1452.

The ring 1454 may also correspond to an intravascular image showing a calcium deposit at the upper region of the image, as shown by the whitened coloration of the ring 1454 in that region. The ring 1455 may also correspond to an image showing a calcium deposit. The ring 1456, however, may correspond to an image showing no calcium presence, as shown by the uniform dark gray color, similar to the ring 1451.

With the calcium distribution rings of Fig. 14 arranged as shown, the user of the system 100 may more quickly identify the location of a calcium deposit both in a longitudinal direction because multiple rings are shown next to one another like the imaged vessel. In addition, because each individual ring shows generally at which area within the vessel the calcium deposit is located (e.g., in the upper, lower, left, or right side of the vessel), the user of the system 100 may also know the angular location of the calcium deposit across multiple intravascular images.

In some embodiments, an indicator, such as the indicator 1460, may identify for the user any rings corresponding to the presence of calcium. This may assist the user of the system 100 in quickly identifying the length of any calcium deposits present within the vessel. As shown, multiple additional calcium composition rings may be included within the illustration shown. For example, a calcium composition ring may be generated associated with each intravascular image received by the processor 510 (Fig. 5).

Fig. 15 is a diagrammatic view of an in-line digital (ILD) display 1500, according to aspects of the present disclosure. In some embodiments, the graphical user interface displayed to a user of the system 100 may include an ILD 1500. The ILD 1500 may include several graphical representations of image frames each associated with an overall frame score 1560.

In some embodiments, the ILD 1500 may assist a user of the system 100 to identify where along the imaged vessel an IVUS image was obtained. The ILD 1500 may be generated by the system 100 based on the IVUS images received by the device 102 (Fig. 1). In some applications, the ILD 1500 may provide a longitudinal cross-sectional view of the blood vessel. In contrast, an IVUS image is a tomographic or radial cross-sectional view of the blood vessel. The ILD 1500 can be a stack of the intraluminal images acquired at various positions along the vessel, such that the longitudinal view of the ILD 1500 is perpendicular to the radial cross-sectional view of the intraluminal image. In such an embodiment, the ILD 1500 may show the length of the vessel, whereas an individual intraluminal image is a single radial cross-sectional image at a given location along the length. In another embodiment, the ILD 1500 may be a stack of the intraluminal images acquired over time during the imaging procedure and the length of the ILD 1500 may represent time or duration of the imaging procedure. The ILD 1500 may be generated and displayed in real time or near real time during the pullback procedure. As each additional intraluminal image is acquired by the device 102 (Fig. 1), it may be added to the ILD 1500. For example, at a point in time during the pullback procedure, the ILD 1500 may be partially complete. In some embodiments, the processor circuit may generate an illustration of a longitudinal view of the vessel being imaged based on the received IVUS images. For example, rather than displaying actual vessel image data as the ILD 1500 does, the illustration may be a stylized version of the vessel, with e.g., continuous lines showing the lumen border and vessel border. For example, the ILD 1500 can be a visualization of the vessel with IVUS image data or can be a stylized illustration of vessel based on the IVUS image data. The ILD 1500 may include an indicator identifying the location along the ILD 1500 at which an IVUS image currently displayed was obtained.

In some embodiments, as shown in Fig. 15, the ILD 1500 may include graphical representations of various colors or patterns corresponding to the overall frame score 1560 of each intravascular image obtained. As explained with reference to Fig. 12, in some embodiments, the processor circuit 510 may be configured to generate a frame score 1292 (Fig. 12) for each received intravascular image. As shown in Fig. 15, a graphical representation may be generated of a particular color associated with that frame score 1560. For example, a graphical representation of a frame 1550 is shown in Fig. 15. The graphical representation of frame 1550 is illustrated in a dark gray color. The processor circuit may define this color to be one associated with a frame with no calcium presence. An overall frame score 1560 may be generated and displayed above the graphical representation of the frame 1550 to also show that no calcium is present within the vessel at frame 1550. Similarly, frames 1551 and 1552 may be of the same color and of either the same frame scores 1560, or frame scores within a predetermined range.

The arrow 1590 may indicate a proximal direction and the arrow 1592 may indicate a distal direction. In this way, the frame 1550 is shown to be the proximal most frame shown in Fig. 15 while the frame 1559 is the distal most frame shown in Fig. 15. In this way, the frames 1553 and 1554, distal of frame 1552, may illustrate a greater calcium content. For example, the overall frame scores 1560 associated with the frames 1553 and 1554 may be higher (or lower depending on how the frame score system has been defined) than the frame scores 1560 of the frames 1550, 1551, and 1552 previously described. The color of the frames 1553 and 1554 may also vary from the frames 1550, 1551, and 1552 may also be changed to reflect the difference in calcium content.

The frames 1555 and 1556 shown distal to the frame 1554 may illustrate frames of even greater calcium content than frames 1553 and 1554. This may be shown by different overall frames scores 1560 and different colors, as shown in Fig. 15.

Finally, the frames 1557, 1558, and 1559 shown distal to the frame 1556 may, like the frames 1550, 1551, and 1552, illustrate intravascular image frames obtained showing no presence of calcium. This may be seen by the same or similar frame scores 1560 as the frames 1550, 1551, and 1552, and/or by the same coloration as the frames 1550, 1551, and 1552.

The ILD 1500 also may identify for a user of the system 100 frames which show calcium presence with an indicator 1572. This may assist the user in quickly and accurately assessing a length measurement of a particular calcium deposit. In addition, in some embodiments, the processor circuit 510 (Fig. 5) may be configured to determine an overall frame score or an average calcium score 1570 associated with all frames shown exhibiting calcium presence. This average calcium score 1570 may be determined in any number of ways. For example, the average calcium score 1570 may include an average of the overall frame scores 1560 of all frames showing calcium presence. In some embodiments, the average calcium score may instead include a maximum of all the overall frame scores 1560, a median, or any other suitable measurement or statistic.

Fig. 16 is a diagrammatic view of a graphical user interface 1600 including an intravascular ultrasound image 1640, an angiography image 1690, and an in-line digital display 1610, according to aspects of the present disclosure. As shown in Fig. 16, the intravascular data, including the intravascular image 1640 along with a calcium distribution ring 1650, and ILD 1610 may be coregistered to the angiography image 1690. The graphical user interface 1600 may include a visual representation of the distance between the calcium and the lumen border (e.g., the calcium depth) and/or a visual representation of the density of the calcium (e.g., the calcium distribution) relative to the other tissue within the vessel. The graphical user interface 1600 may also or alternatively include a numerical or graphical representation of the distance between the calcium and the lumen border (e.g., the calcium depth) and/or a numerical or graphical representation of the density of the calcium (e.g., the calcium distribution) relative to the other tissue within the vessel. The graphical user interface 1600 may include any of the elements shown in any of the figures presented previously or described with reference to any of these figures. The screen display (e.g., the graphical user interface 1600) may include an extraluminal image (e.g., the angiography image 1690) of the blood vessel including the visual representation of the individual numerical score (e.g., the frame score 1692, the scores 1660, the regions 1662, 1664, 1666, or 1668, or the score 1670) or a longitudinal image (e.g., the ILD 1610) of the blood vessel including the visual representation of the individual numerical score (e.g., the regions 1612, 1614, 1616, or 1618).

The angiography image 1690 with co-registered intravascular image 1640 may be displayed to a user in any suitable format. For example, as shown in Fig. 16, the angiography image 1690 may be displayed adjacent to the corresponding intravascular image 1640. The intravascular image 1640 may be an IVUS image. In other embodiments, co-registered intravascular data may include any other suitable images, metrics, or other data and may be overlaid over the angiography image 1690 or arranged or displayed in any other suitable configuration. Such an overlay 1630 is shown overlaid over the IVUS image 1640 in Fig. 16. The screen display (e.g., the graphical user interface 1600) includes a graphical overlay 1630 overlaid on the IVUS image 1640 and the graphical overlay 1630 provides a visual representation of the calcium 1622. The processor circuit may be configured to adjust a transparency of the graphical overlay.

In the embodiment shown in Fig. 16, an indicator 1680 is positioned over the angiography image 1690 at a location along a vessel imaged by the intravascular device 102 (Fig. 1). The IVUS image 1640 displayed adjacent to the angiography image 1690 is an image acquired by the intravascular device at the location identified by the indicator 1680. In some embodiments, a user of the system 100 may also select an additional IVUS image to be displayed in the graphical user interface 1600. As a different IVUS image is selected, the indicator 1680 would move to a different location along the vessel corresponding to the location at which the selected IVUS image was obtained. In some embodiments, a user of the system 100 may additionally move the indicator 1680 along any vessel shown in the angiography image 1690 and an IVUS image corresponding to the selected location would be displayed to the user if an IVUS image is available.

In some embodiments, additional images may be included and displayed to a user of the system 100, including the image longitudinal display (ILD) 1610. The ILD 1610 may provide the user with a longitudinal view of the vessel imaged with the intravascular device. Aspects of the graphical user interface 1600, such as the ILD 1610 may include some features similar to those described in U.S. Patent Publication No. US20200129158A1, titled "GRAPHICAL LONGITUDINAL DISPLAY FOR INTRALUMINAL ULTRASOUND IMAGING AND ASSOCIATED DEVICES, SYSTEMS, AND METHODS," and filed October 24, 2019, which is hereby incorporated by reference in its entirety. Specifically, one end of the ILD 1610 may correspond to the proximal most region of the imaged vessel and the opposing end of the ILD 1610 may correspond to the distal most region of the imaged vessel. The ILD 1610 may provide a visual representation (e.g., numerical/alphanumerical, graphical, symbolic, etc.) of relative diameters of the imaged vessel at all positions along the imaged vessel. The ILD 1610 may include an indicator 1620. The indicator 1620 may correspond to the position of the intravascular device relative to the entire imaged vessel at the location at which the displayed IVUS image was obtained. In this way, as the indicator 1680 is moved by a user to a different location along the vessel, a different IVUS image would be displayed adjacent to the angiography image 1690 and the indicator 1620 would also move to a different corresponding position within the ILD 1610. In some embodiments, a user may be able to move the indicator 1620 to a different location within the ILD 1610 as well and cause the system 100 to recall and display an associated IVUS image as well as move the indicator 1680 to a different corresponding position within the angiography image 1690.

As shown in Fig. 16, the intravascular image 1640 may also include a calcium distribution ring 1650. In addition, processor circuit 510 (Fig. 5) may generate an overall frame score 1692 associated with the intravascular image 1640. This frame score 1692 may be displayed in any suitable location relative to the intravascular image 1640 or any other element of the graphical user interface 1600. The frame score 1692 may be a numerical representation shown above the IVUS image 1640. In some embodiments, the frame score 1692 may be displayed as a visual representation or a graphical representation. For example, the frame score 1692 may additionally or alternatively be displayed as shade of a color corresponding to the score, a pattern, a size, or by any other means.

The ILD 1610 may additionally include several colored region on either side of the ILD 1610. For example, regions 1612, 1614, 1616, and 1618 are shown adjacent to the ILD 1610. These regions may correspond to locations within the vessel of the same or similar overall frame scores. For example, like the frames shown in Fig. 15, in which each frame of is colored according to the overall frame score indicating to the user the extent of calcium deposits shown in frame, each location or IVUS image used to create the ILD 1610 may similarly be associated with a frame score. The color, or in some embodiments, the pattern, of the region 1612 may correspond to this frame score. For example, the IVUS images obtained along the region 1612 of the ILD 1610 may have been assigned the same or similar frame scores, and are thus grouped into a single region 1612 identified on either side of the ILD 1610. As an example, the region 1612 may refer to IVUS images which do not depict calcium deposits. The color and/or pattern the regions 1662, 1664, 1666, and 1668 can similarly correspond to the frame score.

The region 1614, adjacent to the region 1612 may similarly identify IVUS images with the same or similar frame scores as each other. For example, these images may depict a greater presence of calcium deposits. The region 1616 adjacent to the region 1614 may correspond to images of even greater presence of calcium deposits. And finally, the region 1618 may be associated with regions of similar extents of calcium deposits as the region 1612, as denoted by the similar visual appearance of the indicators on either side of the ILD 1610.

An overlay is observed in the angiography image 1690. This overlay includes regions corresponding to the regions 1612, 1614, 1616, and 1618 of the ILD 1610. For example, a region 1662 is observed overlaid over a portion of the vessel shown in the angiography image 1690. This region 1662, like the region 1612, may have been assigned the same or similar frame scores, and are thus grouped into a single region 1662 identified. This region 1662 may show to the user where along the vessel the IVUS images showing no calcium deposits are located. Similarly, the region 1664 of the angiography image 1690 may be associated with the region 1614 of the ILD and may show to a user where along the vessel within the angiography image 1690 the images used to generate the section of the ILD 1610 corresponding to the region 1614 are located. The region 1666 of the angiography image 1690 may be similarly associated with the region 1616 of the ILD 1610 and the region 1668 of the angiography image 1690 may be associated with the region 1618 of the ILD 1610.

Also shown overlaid over the angiography image 1690 may be an indicator 1672. This indicator may identify for the user of the system 100 regions along the vessel within the angiography image where calcium deposits were measured. Additionally, frame scores 1660 may be provided within the angiography image. These frame scores 1660 may indicate a common or average frame score of all IVUS images combined into their corresponding regions. In addition, an average score 1670 may also be generated corresponding to the region where calcium deposits were detected. The score 1670 may be similar to the score 1570 described with reference to Fig. 15.

It is noted that the angiography image 1690, in some embodiments, may be or include a graphical representation generated by the processor circuit 510 (Fig. 5). For example, the image 1690 may be based on an angiography image but may be generated in a diagrammatic form to simplify the display. In other embodiments, the angiography image 1690 may be an actual angiography image with an overlay including the regions 1662, 1664, 1666, and 1668 as well as any other indicators or measurements.

In some embodiments, the processor circuit can generate and output treatment recommendations based on the calculated frame scores. For example, ranges of frame scores can be associated with different levels of severity of the blockage (e.g., the nature/extent of the calcium forming the blockage). For example, if the frame scores have a value between 1-10 and there are three different levels of severity (levels 1, 2, and 3), then frame scores 2-4 can be associated with level 1 (the relatively lowest severity), frame scores 5-7 can be associated with level 2 (relative severity between level 1 and level 2), and frame scores 8-10 can be associated with level 3 (the relatively highest severity). A frame score of 1 may indicative some concern, but not enough to warrant intervention. In some embodiments, the screen display includes a visual representation of the level of severity, e.g., in lieu of or in addition to the frame score 1692. For example, the coloring and/or pattern of the regions 1612, 1614, 1618, 1618, 1662, 1664, 1666, and/or 1668 can correspond to the level of severity. In some embodiments, a numerical/textual description of the level of severity is provided on the screen display 1600. The processor circuit can output a therapy recommendation based on the level of severity and/or the frame score. A less aggressive therapy recommendation can be made for a lower level of severity and/or frame score, and a more aggressive therapy recommendation can be made for a higher level of severity and/or frame score. For example, for a relatively lower severity (level 1) and/or frame scores (frame scores 2-4 on a 1-10 scale), the processor circuit can output a recommendation to use a scoring balloon. For a relatively moderate severity (level 2) and/or frame scores (frame scores 5-7 on a 1-10 scale), the processor circuit can output a recommendation to use atherectomy (manual cutting with blade/cutter, laser, etc.) and/or aspiration. For a relatively higher severity (level 3) and/or frame scores (frame scores 8-10 on a 1-10 scale), the processor circuit can output a recommendation to use rotational atherectomy (e.g., Rotoblator™ available from Boston Scientific). The therapy recommendation can be part vessel preparation planning that is completed before stenting the vessel. In that regard, vessel preparation can include removing calcium and/or thrombus from the vessel before the stent is placed. In that regard, calcium may crumble relatively easy when pressure (e.g., from a stent being expanded) is applied. Removing the calcium prior to stenting improves the efficacy of the stenting by ensuring that the stent is properly deployed and has good apposition against the lumen wall.

Any of the aspects, methods, or steps described in the present application may be performed before or after a treatment procedure. In some aspects, treatment procedures may include positioning a stent at a location along the vessel corresponding to a decrease in blood flow as a result of, for example, a lesion or an obstruction. Treatment procedures may also include the deployment of a balloon (e.g., an angioplasty balloon), a graft, a filter, a valve, an atherectomy procedure, a cryotherapy procedure, an ablation procedure, the delivery of a drug, or any other treatment procedure at the location of the reduction in blood flow.

In some embodiments, the processor circuit 510 may associate any of the treatment procedures previously identified with a predicted reduction or change in calcium presence or composition. For example, the deployment of a stent at the site of the obstruction may be predicted to restore blood flow by a predetermine amount or percentage. In some embodiments, the deployment of a stent at the site of obstruction may be associated with a predicted complete restoration of blood flow. In some embodiments, the deployment of a stent may be associated with some lesser degree of restoration of blood flow. Partial restoration of blood flow may be quantified and displayed as a percentage of normal or target blood flow, as a ratio, or by any other means. The deployment of a stent may be associated with a change in the calcium distribution. For example, the processor circuit may associate the deployment of a stent with an increase in the distance of a particular calcium deposit to the center of a lumen by a set amount or percentage. The deployment of a stent may also be associated with a predicted change in comparisons of the cross-sectional area of calcium with a luminal cross-sectional area. Similarly, an atherectomy procedure may be associated with a change in the calcium distribution. For example, an atherectomy procedure may be associated by the processor circuit 510 with a percent reduction in calcium presence at the obstruction site. Any of the predicted changes in calcium presence associated with the various treatment procedures listed may be based on previous treatment procedures, recommendations of experts in the field, predictions input by a user of the system, or any other source.

In some embodiments, the processor circuit may be configured to determine a predicted calcium distribution within the vessel after a treatment procedure has been performed. This treatment procedure may include any of those listed. The processor circuit may be configured to determine a numerical score based on the predicted calcium distribution. This numerical score may be similar to any of the scores described. Such a score may provide the user with rapid and accurate data associated with all possible treatment procedures and may assist a physician in determining the correct method of treatment for each particular patient and obstruction.

In some embodiments, any of the aspects, methods, and steps of the disclosed invention may be performed before or after any of the treatment procedures described. In one aspect, the system may determine numerical scores related to the calcium presence and/or distribution after a treatment procedure has been performed. For example, the processor circuit 510 may receive additional IVUS imaging data from the IVUS imaging catheter after a treatment procedure has been performed. The processor circuit 510 may identify calcium based on the additional IVUS imaging data. The circuit may then calculate, based on the identification of the calcium, an additional individual numerical score representative of the calcium. The circuit may then output, to the screen display, a comparison of the individual numerical score to the additional individual numerical score. In this way, a comparison between a numerical score representative of calcium presence at any particular location of a vessel may be determined before and after treatment. The two scores may be simultaneously displayed to a user. The display and comparison of the two scores may assist a user of the system or a physician to determine the success of the treatment procedure.

In some embodiments, the processor circuit 510 may generate a numerical value representative of a difference in cross-sectional area of the blood vessel before and after the treatment procedure. In other embodiments, this numerical value may represent a difference in cross-sectional area of calcium, other plaque, or other tissue within the vessel before and after the treatment procedure.

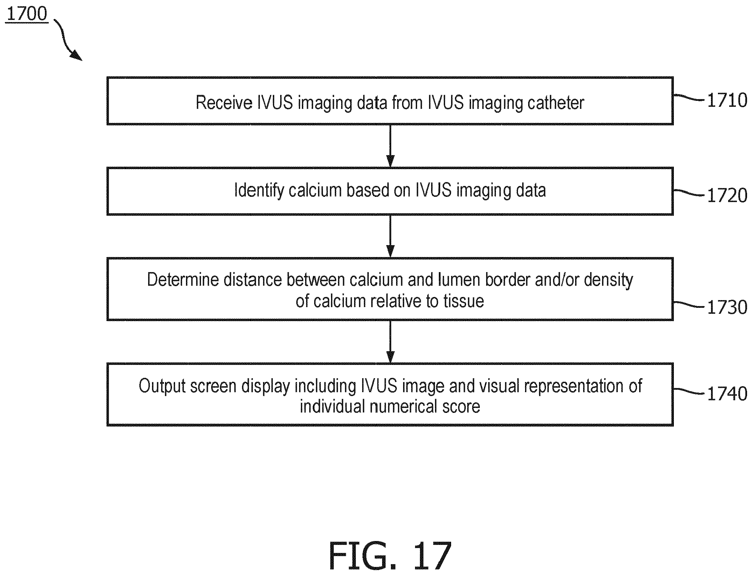

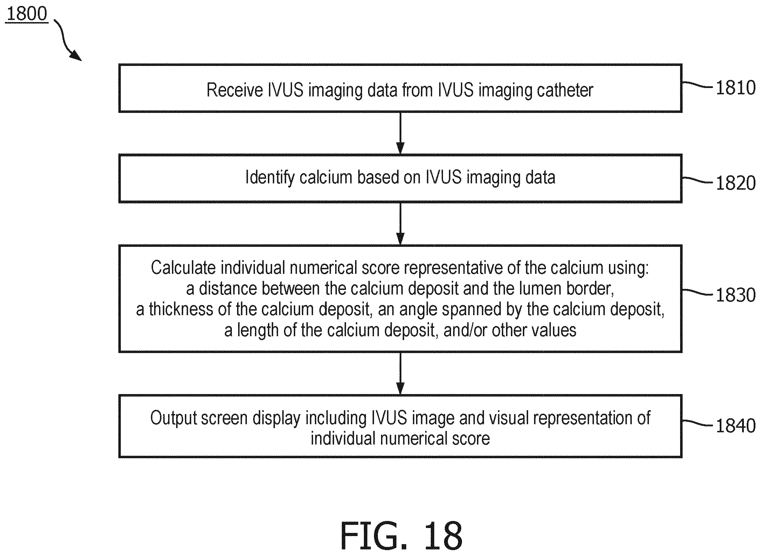

Figs. 17 and 18 are flow diagrams for methods 1700 and 1800, respectively, of identifying the location and extent of calcium within a vessel and conveying associated metrics to a user, according to aspects of the present disclosure. As illustrated, the methods 1700 and 1800 include a number of enumerated steps, but embodiments of the methods 1700 and 1800 may include additional steps before, after, or in between the enumerated steps. In some embodiments, one or more of the enumerated steps may be omitted, performed in a different order, or performed concurrently. The steps of the methods 1700 and 1800 can be carried out by any suitable component within the system 100 and all steps need not be carried out by the same component. In some embodiments, one or more steps of the methods 1700 and 1800 can be performed by, or at the direction of, a processor circuit of the system 100 (e.g., the processor circuit 210 of Fig. 2), including, e.g., the processor 260 or any other component.

Referring now to Fig. 17, at step 1710, the method 1700 includes receiving intravascular data from an intravascular catheter. For example, the processor circuit may receive IVUS imaging data from an IVUS imaging catheter. At step 1720, the method 1700 includes identifying calcium based on the intravascular data using a deep learning network. For example, the processor circuit may identify calcium based on the IVUS imaging data. At step 1730, the method 1700 includes determining, based on the identification of calcium, a distance between the calcium and the lumen border, and/or a density of the calcium relative to tissue. In some embodiments, a quantification of the density of calcium relative to tissue may include a numerical score representative of the calcium. The calculation of the density of calcium may include as inputs the distance between the calcium and lumen border, a length of the calcium longitudinally along the vessel, a cross-sectional surface area of the calcium, and/or a cross-sectional surface area of other tissue. At step 1740, the method 1700 includes outputting a screen display to the display in communication with the processor circuit. The screen display includes an IVUS image generated based on the IVUS imaging data. The screen display also includes a visual representation of the distance and/or a visual representation of the density.

Referring now to Fig. 18, at step 1810, the method 1800 includes receiving intravascular data from an intravascular catheter. For example, the processor circuit may receive IVUS imaging data from an IVUS imaging catheter. At step 1820, the method 1800 includes identifying calcium based on the intravascular data. For example, the processor circuit may identify calcium based on the IVUS imaging data using a deep learning network. At step 1830, the method 1800 includes determining, based on the identification of calcium, an individual numerical score representative of the calcium. For example, the processor circuit can calculate the individual numerical score based on a distance between the calcium deposit and the lumen border, a thickness of the calcium deposit, an angle spanned by the calcium deposit; a length of the calcium deposit, and/or other values. At step 1840, the method 1800 includes outputting a screen display to the display in communication with the processor circuit. The screen display includes an IVUS image generated based on the IVUS imaging data. The screen display also includes a visual representation of the individual numerical score. The individual numerical score advantageously provides a quantity that a physician can more easily use to gauge the relative severity of a blockage of the blood vessel. The calcium forms all or some part of the blockage.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

**Claims**

1. An intravascular ultrasound imaging system (100), comprising:

    an intravascular ultrasound, IVUS, imaging device (102) configured to obtain IVUS imaging data while positioned within a blood vessel of a patient;
    a processor circuit (134) configured to:

        receive the IVUS imaging data (710) from the IVUS imaging device;
        identify calcium (1020, 1023) that is comprised by the blood vessel based on the IVUS imaging data;

determine, based on the identification of the calcium, at least one of:

> a distance (1044) between the calcium (1023) and a lumen border (1032) defining a lumen of the blood vessel; or
> a density of the calcium relative to tissue forming the wall of the blood vessel;

output to a display (132) an IVUS image generated based on the IVUS imaging data and at least one of:

> a visual representation of the distance; or
> a visual representation of the density.

2. The system of claim 1, wherein the processor circuit is configured to use a deep learning network (724) to identify the calcium, and wherein preferably, the processor circuit is configured to use the deep learning network to determine at least one of the distance or the density.

3. The system of claim 2,

> wherein the deep learning network is trained using a plurality of annotated IVUS images, and
> wherein each of the plurality of annotated IVUS images comprises an annotation identifying the calcium.

4. The system of claim 1, wherein the distance comprises a radial distance.

5. The system of claim 1, wherein the processor circuit is configured to determine the density for at least one of a scan line of the IVUS image, a sector of the IVUS image, an entirety of the IVUS image, or a plurality of IVUS images.

6. The system of claim 1,

> wherein the screen display further comprises a graphical overlay overlaid on the IVUS image;
> wherein the graphical overlay provides a visual representation of the calcium, and
> wherein preferably, the processor circuit is configured to adjust a transparency of the graphical overlay.

7. The system of claim 6,

> wherein the graphical overlay provides a visual representation of a plurality of tissue types other than calcium, and
> wherein the plurality of tissue types other than calcium are grouped together such that the graphical overlay only includes the visual representation of the calcium and the visual representation of the plurality of tissue types other than calcium.

8. The system of claim 1, wherein the processor circuit is configured to determine an individual numerical score representative of the calcium, wherein the individual numerical score is based on at least one of the distance or the density.

9. An intravascular ultrasound imaging system (100), comprising:

> an intravascular ultrasound, IVUS, imaging device (102) configured to obtain IVUS imaging data while positioned within a blood vessel of a patient;
> a processor circuit (134) configured to:
>
> > receive the IVUS imaging data (710) from the IVUS imaging device;
> > identify calcium (1020, 1023) that is comprised by the blood vessel based on the IVUS imaging data;
> > calculate, based on the identification of the calcium, an individual numerical score (1260, 1292) representative of the calcium using:
> >
> > > a distance between the calcium deposit and the lumen border;
> > > a thickness of the calcium deposit;
> > > an angle spanned by the calcium deposit; and
> > > a length of the calcium deposit; and

output to a display (132) an IVUS image generated based on the IVUS imaging data and a visual representation of the individual numerical score.

10. The system of claim 9, wherein processor circuit is configured to use a deep learning network to identify the calcium and wherein preferably, the processor circuit is configured to use the deep learning network to determine the distance, the thickness, the angle, and the length.

11. The system of claim 9, wherein the distance, the thickness, the angle, and the length are weighted based on a location of the calcium within the wall.

12. The system of claim 9, wherein the individual numerical score corresponds to at least one of a scan line of the IVUS image, a sector of the IVUS image, an entirety of the IVUS image, or a plurality of IVUS images.

13. The system of claim 9,
wherein the screen display further comprises at least one of:

an extraluminal image of the blood vessel including the visual representation of the individual numerical score; or
a longitudinal image of the blood vessel including the visual representation of the individual numerical score.

14. The system of claim 9, wherein the processor circuit is further configured to:

determine a predicted calcium distribution within the vessel after a treatment procedure has been performed; and
generate a predicted numerical score based on the predicted calcium distribution.

15. The system of claim 9, wherein the processor circuit is further configured to:

receive additional IVUS imaging data from the IVUS imaging device after a treatment procedure has been performed;
identify calcium based on the additional IVUS imaging data;
calculate, based on the identification of the calcium, an additional individual numerical score representative of the calcium; and
output, to the screen display, a comparison of the individual numerical score to the additional individual numerical score, and

wherein preferably, the comparison comprises a numerical value representative of a difference in cross-sectional area of the blood vessel before and after the treatment procedure.

**FIG. 1**

EP 4 201 342 A1

FIG. 2

EP 4 201 342 A1

FIG. 3

EP 4 201 342 A1

FIG. 4

EP 4 201 342 A1

FIG. 5

FIG. 6

FIG. 7

EP 4 201 342 A1

FIG. 8

FIG. 9

FIG. 10

FIG. 11

FIG. 12

FIG. 13

FIG. 14

1500

1590

1570

1572

1592

1560

## ## ## ## ## ## ## ## ## ##

. . . . . .

1550 1551 1552 1553 1554 1555 1556 1557 1558 1559

FIG. 15

FIG. 16

<u>1700</u>

```
┌─────────────────────────────────────────────────────────────┐
│      Receive IVUS imaging data from IVUS imaging catheter      │───1710
└─────────────────────────────────────────────────────────────┘
                              │
                              ▼
┌─────────────────────────────────────────────────────────────┐
│           Identify calcium based on IVUS imaging data          │───1720
└─────────────────────────────────────────────────────────────┘
                              │
                              ▼
┌─────────────────────────────────────────────────────────────┐
│  Determine distance between calcium and lumen border and/or density  │
│              of calcium relative to tissue                     │───1730
└─────────────────────────────────────────────────────────────┘
                              │
                              ▼
┌─────────────────────────────────────────────────────────────┐
│  Output screen display including IVUS image and visual representation of  │
│              individual numerical score                        │───1740
└─────────────────────────────────────────────────────────────┘
```

FIG. 17

```
┌─────────────────────────────────────────────────────────────┐
│     Receive IVUS imaging data from IVUS imaging catheter      │──1810
└─────────────────────────────────────────────────────────────┘
                              │
                              ▼
┌─────────────────────────────────────────────────────────────┐
│           Identify calcium based on IVUS imaging data          │──1820
└─────────────────────────────────────────────────────────────┘
                              │
                              ▼
┌─────────────────────────────────────────────────────────────┐
│ Calculate individual numerical score representative of the calcium using: │
│         a distance between the calcium deposit and the lumen border,       │──1830
│ a thickness of the calcium deposit, an angle spanned by the calcium deposit, │
│          a length of the calcium deposit, and/or other values              │
└─────────────────────────────────────────────────────────────┘
                              │
                              ▼
┌─────────────────────────────────────────────────────────────┐
│ Output screen display including IVUS image and visual representation of │──1840
│                    individual numerical score                  │
└─────────────────────────────────────────────────────────────┘
```

FIG. 18

EP 4 201 342 A1

Europäisches
Patentamt

European
Patent Office

Office européen
des brevets

## EUROPEAN SEARCH REPORT

Application Number

EP 22 16 8632

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/294659 A1 (GOPINATH AJAY [US] ET AL) 17 September 2020 (2020-09-17) | 1-12,14,15 | INV. A61B8/08 A61B8/12 |
| Y | * paragraph [0077] * <br> * paragraph [0080] * <br> * paragraph [0097] * <br> * paragraph [0119] * <br> * paragraph [0120] * <br> * paragraph [0112] * <br> * figure 2D * | 13 | |
| X | WO 2021/062006 A1 (BOSTON SCIENT SCIMED INC [US]) 1 April 2021 (2021-04-01) | 1-3 | |
| Y | * paragraph [0121] * <br> * paragraph [0127] * <br> * paragraph [0096] * <br> * paragraph [0111] – paragraph [0113] * <br> * paragraph [0128] * | 13 | |

| TECHNICAL FIELDS SEARCHED (IPC) |
|---|
| A61B |

The present search report has been drawn up for all claims

| Place of search | Date of completion of the search | Examiner |
|---|---|---|
| Munich | 23 September 2022 | Ordavo, Ivan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 8632

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2022

| Patent document cited in search report | | Publication date | Patent family member(s) | | Publication date |
|---|---|---|---|---|---|
| US 2020294659 | A1 | 17-09-2020 | CA | 3133449 A1 | 24-09-2020 |
| | | | CN | 113614845 A | 05-11-2021 |
| | | | EP | 3941335 A2 | 26-01-2022 |
| | | | JP | 2022525469 A | 16-05-2022 |
| | | | US | 2020294659 A1 | 17-09-2020 |
| | | | WO | 2020190976 A2 | 24-09-2020 |
| WO 2021062006 | A1 | 01-04-2021 | CN | 114727802 A | 08-07-2022 |
| | | | EP | 4033988 A1 | 03-08-2022 |
| | | | WO | 2021062006 A1 | 01-04-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7846101 B
- US 6776763 B
- US 7226417 B
- US 61985220
- US 6200268 B, D. Geoffrey Vince, Barry D. Kuban and Anuja Nair
- US 6381350 B, Jon D. Klingensmith, D. Geoffrey Vince and Raj Shekhar
- US 7074188 B, Anuja Nair, D. Geoffrey Vince, Jon D. Klingensmith and Barry D. Kuban
- US 7175597 B, D. Geoffrey Vince, Anuja Nair and Jon D. Klingensmith
- US 7215802 B, Jon D. Klingensmith, Anuja Nair, Barry D. Kuban and D. Geoffrey Vince
- US 7359554 B, Jon D. Klingensmith, D. Geoffrey Vince, Anuja Nair and Barry D. Kuban
- US 7463759 B, Jon D. Klingensmith, Anuja Nair, Barry D. Kuban and D. Geoffrey Vince
- US 20200129158 A1